# EUROPÄISCHE PATENTANMELDUNG

(11) **EP 4 122 591 A1**
(43) Veröffentlichungstag der Anmeldung: **25.01.2023**
(21) Anmeldenummer: 21020381.6
(22) Anmeldetag: 23.07.2021
(51) Int. Cl.: B01J 8/06, C07C 11/04, C07C 51/16, B01J 8/00

(54) **VERFAHREN UND ANLAGE ZUR HERSTELLUNG EINER ZIELVERBINDUNG**

(71) Anmelder: Linde GmbH, 82049 Pullach (DE)
(72) Erfinder: Meiswinkel, Andreas, 82049 Pullach (DE); Wöhl, Anina, 82049 Pullach (DE); Zellhuber, Mathieu, 82049 Pullach (DE); Schubert, Martin, 82049 Pullach (DE)

(57) **Zusammenfassung**

Die vorliegende Erfindung schlägt ein Verfahren zur Herstellung einer Zielverbindung vor, bei dem ein wenigstens eine Eduktverbindung in Form eines oder mehrerer Kohlenwasserstoffe enthaltendes Einsatzgemisch (A) gebildet, auf parallel angeordnete Reaktionsrohre (10) eines oder mehrerer Rohrbündelreaktoren (100) verteilt, und in den Reaktionsrohren (10) einer oxidativen katalytischen Umsetzung unterworfen wird. Es ist vorgesehen, dass Überwachungsmaßnahmen durchgeführt werden, die umfassen, auf Grundlage von Temperaturmessungen an mehreren Messstellen entlang eines oder mehrerer der Reaktionsrohre (10) jeweils zeitliche Temperaturverläufe zu ermitteln, auf Grundlage zumindest der Temperaturverläufe eine Stabilitätsbewertung der katalytischen Umsetzung vorzunehmen, und, wenn auf Grundlage der Stabilitätsbewertung ein instabiler Zustand der katalytischen Umsetzung als eingetreten oder wahrscheinlich eintretend bestimmt wird, Stabilisierungsmaßnahmen einzuleiten. Eine entsprechende Anlage ist ebenfalls Gegenstand der Erfindung.

## Beschreibung

Die vorliegende Erfindung betrifft ein Verfahren und eine Anlage zur Herstellung einer Zielverbindung gemäß den Oberbegriffen der entsprechenden unabhängigen Patentansprüche.

### Hintergrund der Erfindung

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den jeweiligen Olefinen und zu Wasser umgesetzt. Die vorliegende Erfindung betrifft insbesondere die oxidative Dehydrierung von Ethan zu Ethylen, nachfolgend auch als ODHE bezeichnet. Die vorliegende Erfindung ist grundsätzlich jedoch nicht auf die oxidative Dehydrierung von Ethan beschränkt, sondern kann sich auch auf die oxidative Dehydrierung (ODH) anderer Paraffine wie Propan oder Butan erstrecken. Die nachfolgenden Erläuterungen gelten in diesem Fall entsprechend.

Die ODH(E) kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen und der praktisch irreversiblen Wasserbildung keine thermodynamische Gleichgewichtslimitierung. Die ODH(E) kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine in-situ-Regenerierung ermöglicht bzw. bewirkt. Schließlich werden im Gegensatz zum Steamcracken geringere Mengen an wertlosen Nebenprodukten wie Koks gebildet.

Zu weiteren Details bezüglich der ODH(E) sei auf einschlägige Fachliteratur, beispielsweise Ivars, F. und López Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, sowie X. Li, E. Iglesia, Kinetics and Mechanism of Ethane Oxidation to Acetic Acid on Catalysts Based on Mo-V-Nb Oxides, J. Phys. Chem. C, 2008, 112, 15001-15008, verwiesen.

Insbesondere haben sich für die ODH(E) MoVNb-basierte Katalysatorsysteme als vielversprechend herausgestellt, wie beispielsweise bei F. Cavani et al., "Oxidative dehydrogenation of ethane and propane: How far from commercial implementation?", Catal. Today, 2007, 127, 113-131, erwähnt. Auch zusätzlich Te enthaltende Katalysatorsysteme können verwendet werden. Ist hier von einem "MoVNb-basierten Katalysatorsystem" oder einem "MoVTeNb-basierten Katalysatorsystem" die Rede, sei hierunter ein Katalysatorsystem verstanden, das die genannten Elemente als Mischoxid aufweist, auch ausgedrückt mit MoVNbOₓ bzw. MoVTeNbOₓ. Die Angabe von Te in Klammern steht für dessen optionale Anwesenheit. Die Erfindung kommt insbesondere mit solchen Katalysatorsystemen zum Einsatz.

Bei der ODH werden insbesondere bei Verwendung von MoVNb(Te)Oₓ-basierten Katalysatoren unter industriell relevanten Reaktionsbedingungen signifikante Mengen der jeweiligen Carbonsäuren der eingesetzten Paraffine, im Falle der ODHE insbesondere Essigsäure, als Nebenprodukte gebildet. Für einen wirtschaftlichen Anlagenbetrieb ist daher eine Koppelproduktion von Olefinen und der Carbonsäuren bei Verwendung des beschriebenen Katalysatortyps in der Regel unvermeidlich, eine bevorzugte Bildung von Olefinen allerdings wünschenswert.

Die vorliegende Erfindung und ihre Hintergründe werden nachfolgend mit einem Schwerpunkt auf der ODH(E) beschrieben. Die vorliegende Erfindung eignet sich jedoch grundsätzlich auch zur Verwendung im Zusammenhang mit anderen oxidativen Verfahren zur Umsetzung von Kohlenwasserstoffen, in denen die nachfolgend erläuterten Probleme ebenfalls auftreten können.

Oxidative Verfahren, insbesondere also auch Verfahren zur Umsetzung von Kohlenwasserstoffen mit Sauerstoff, verlaufen hinsichtlich ihrer Wärmetönung exotherm, d.h. sie setzen Wärme frei. Demzufolge ist die oxidative Dehydrierung von Alkanen zu Alkenen und den entsprechenden Carbonsäuren ebenfalls exotherm. Im Falle der ODHE werden, wie eingangs erwähnt, als Wertprodukte Ethylen und Essigsäure gebildet, als Nebenprodukte entstehen in geringen Mengen auch die Totaloxidationsprodukte Kohlenmonoxid und Kohlendioxid. Die Exothermie steigt dabei mit steigendem Oxidationsgrad der Produkte an. So beträgt die Standardreaktionsenthalpie der Reaktion, jeweils ausgehend vom Ethan mit Sauerstoff, zu den Produkten (bzw. Nebenprodukten) Ethylen, Essigsäure, Kohlenmonixid und Kohlendioxid -105 kJ/mol, -590 kJ/mol, -862 kJ/mol und -1.428 kJ/mol. Die absolute Wärmetönung der Gesamtumsetzung ist dabei abhängig von der summierten Ethankonversion und der Selektivität der einzelnen Teilreaktionen.

Eine großtechnische Umsetzung solcher exothermer Verfahren bedarf daher einer effizienten Kontrolle und Abfuhr der Reaktionswärme, um ein thermisches Durchgehen des Reaktors, also einen unkontrollierten, beschleunigten Temperaturanstieg im Katalysatorbett der Reaktionsrohre (siehe zur Ausgestaltung entsprechender Reaktionen sogleich) durch plötzliche Steigerung der Reaktionsgeschwindigkeit und/oder Änderung der Reaktionsselektivität hin zur Totaloxidation zu vermeiden und somit einen sicheren Anlagenbetrieb zu gewährleisten.

In großtechnischen Oxidationsprozessen von Kohlenwasserstoffen kommen häufig Festbettreaktoren zum Einsatz, in denen ein heterogener Katalysator in ein Reaktorvolumen eingefüllt ist und von den Reaktanden überströmt wird. Für exotherme Oxidationsprozesse sind diese Festbettreaktoren in der Regel als gekühlte Rohrbündelreaktoren ausgeführt, bei denen sich eine Vielzahl (üblicherweise mehrere tausend bis mehrere zehntausend) Reaktionsrohre in einem Reaktor befinden. Diese Reaktionsrohre werden bei diesen exothermen Verfahren von einem Kühlmedium, z.B. einer flüssigen Salzschmelze oder einem Thermoöl, umströmt, um die Reaktionswärme abzuführen. Die entstehende Reaktionswärme wird also zumindest zum Teil an das entsprechende Kühlmedium abgegeben. Um das Kühlmittel seinerseits auf einem geeigneten Temperaturniveau zu halten, wird dieses wiederum gegen Dampf gekühlt bzw. die überschüssige Wärme wird genutzt, um damit Dampf zu erzeugen, der an einer anderen Stelle im (großtechnischen, d.h. andere Reaktionsschritte, Trennschritte und dgl. einschließenden) Prozess benötigt wird.

Bei den erwähnten Rohrbündelreaktoren befinden sich am Eintritt und Austritt jeweils eine alle Reaktionsrohre abdeckende Haube, wobei der Reaktionseintrittsstrom über die reaktoreintrittsseitige Haube dem Reaktor zugeführt und entsprechend gleichmäßig auf alle Rohre verteilt wird und der Prozessgasstrom über die reaktoraustrittsssitige Haube aus jedem Reaktionsrohr wieder in einen einzigen Prozessgasstrom vereinigt und den stromab befindlichen Prozesseinheiten zugeführt wird. Ein derartiger gekühlter Rohrbündel-Festbettreaktor (nachfolgend auch kurz als Rohrbündelreaktor, Festbettreaktor oder auch nur als Reaktor bezeichnet) ist aufgrund seiner relativen Einfachheit hinsichtlich der Katalysatorhandhabung (Festbett) sowie der Dimensionierung und des Wärmemanagements auch für Verfahren wie die ODH(E) das bevorzugte Reaktordesign.

Zur Überwachung und Steuerung solcher Rohrbündelreaktoren sind diese Reaktoren mit einer repräsentativen Anzahl an Instrumentierungen, insbesondere zur Temperaturüberwachung, sowohl auf der Kühlmittelseite (z.B. auf Seiten der Salzschmelze) als auch insbesondere auf der Prozessseite, d.h. im Katalysatorbett der Reaktionsrohre, ausgestattet. Allgemein wird hier unter einer "Instrumentierung" die Ausstattung eines Reaktors mit Sensoren bzw. Messeinrichtungen verstanden. Insbesondere die Termperaturüberwachung im Katalysatorbett dient der Überwachung des axialen Temperaturprofils in Flussrichtung entlang der Reaktionsrohre. Die Instrumentierung, d.h. die Ausstattung der Rohre wie auch der Kühlmittelseite mit Temperaturüberwachung, wird gemäß Stand der Technik i.d.R. insbesondere mittels Multipunkt-Thermoelementen oder mittels Einzelpunkt-Thermoelementen (engl. multipoint thermocouples, single-point thermocouples) ausgeführt.

Multipunkt-Thermoelemente sind Thermoelemente, die in einem vorgegebenen Abstand (i.d.R. aber nicht zwingend äquidistant) Temperaturmesspunkte besitzen, so dass das axiale Temperaturprofil in Flussrichtung des Katalysatorbetts bereits in einem einzigen Rohr mit einer gewissen Anzahl (z.B. 5 bis 15) an Stützstellen (entspricht Anzahl der Temperaturmesspunkte des Multipunkt-Thermoelements) ermittelt werden kann. Es sei hier erwähnt, dass auch faseroptische Messverfahren zur Temperaturmessung bekannt sind. Diese faseroptischen Systeme liefern ähnlich wie die Multipunkt-Thermoelemente ein Profil bereits in einem einzigen Rohr (i.d.R allerdings mit einer deutlich größeren Auflösung, d.h. einer deutlich größeren Anzahl an Stützstellen bis hin zu einem nahezu kontinuierlichen Profil). Faseroptische Messsysteme eignen sich also (ähnlich wie Multipunkt-Thermoelemente) auch in Sinne der Erfindung zur Ermittelung der axialen Temperaturprofile. Einzelpunkt-Thermoelemente dagegen weisen genau nur einen Temperaturmesspunkt (an der Spitze der jeweiligen Thermoelemente) auf. Zur Bestimmung eines in Flussrichtung axialen Temperaturprofils wird demnach vorteilhafterweise eine gewisse Anzahl an Reaktionsrohren (z.B. 5 bis 15 Rohre) an unterschiedlichen Positionen im Katalysatorbett mit einem solchen Einzelpunkt-Thermoelement ausgestattet, wobei sich diese Rohre hinsichtlich der Temperaturverteilung vorteilhafterweise auf der Kühlmittelseite in einem zumindest ähnlichen bzw. gleichen Bereich befinden.

In einem großtechnischen Reaktor mit mehreren 1000 oder mehreren 10000 Rohren wird eine gewisse Anzahl an Rohren so mit einer Temperaturüberwachung ausgestattet, dass eine für den Reaktor repräsentative Anzahl an in Flussrichtung axialen Katalysatortemperaturprofilen erhalten wird. Insbesondere können so verschiedene radial verteilte Bereiche eines großtechnischen Rohrbündelreaktors überwacht werden, da auch die Kühlmitteltemperatur einer radialen Verteilung unterliegt.

Bei dieser Vorgehensweise ist klar, dass im Falle der Instrumentierung mit Einzelpunkt-Thermoelementen, um eine Anzahl N in Flussrichtung axiale Temperaturprofile zu erhalten, eine Anzahl N x M Rohre instrumentiert werden müssen, damit jedes Temperaturprofil M Stützstellen enthält.

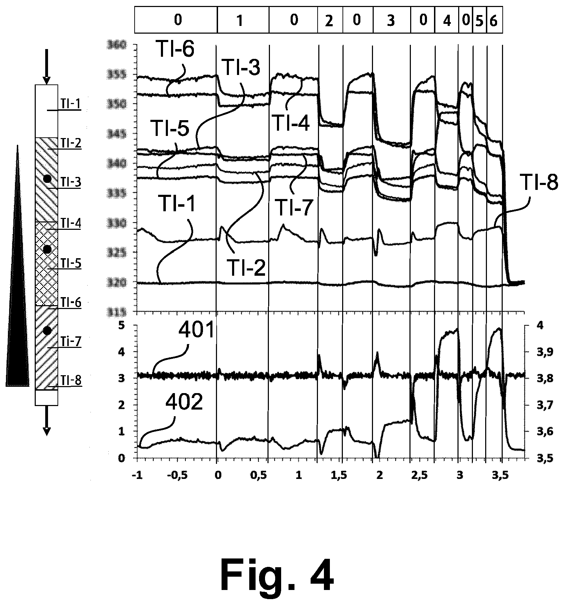

Zu weiteren Details bezüglich entsprechender Reaktoren sei auf einschlägige Fachliteratur wie beispielsweise den Artikel von G. Eigenberger, "Fixed-Bed Reactors", in Ullmann's Encyclopedia of Industrial Chemistry, Band B 4, 1992, Seiten 199 bis 238, verwiesen. Wie dort beispielsweise im Zusammenhang mit Figur 4.1 D und E beschrieben, ist zur Temperaturführung in Rohrbündelreaktoren auch die Verwendung mehrerer Kühl- und Reaktionszonen bekannt.

Die Verwendung eines entsprechenden mehrzonigen Reaktors ist für stark exotherme Reaktionen, insbesondere auch für oxidative Reaktionen wie die ODH(E), beispielsweise auch in der WO 2019/243480 A1 beschrieben. Wie dort offenbart, können dabei unterschiedliche Katalysatorbetten bzw. Reaktionszonen, die unterschiedliche Katalysatorbeladungen und/oder Katalysatoraktivitäten pro Raumeinheit aufweisen, zum Einsatz kommen. Dadurch wird der für solch exotherme Reaktionen typische Hotspot entlang des Katalysatorbetts gestreckt und die absolute Temperaturdifferenz vom Hotspot zur Kühlmitteltemperatur bzw. vom Ende einer vorausgehenden Reaktionszone zum Hotspot der darauffolgenden Reaktionszone verringert, wodurch auch die Gefahr eines thermischen Durchgehens des (gekühlten) Reaktors - bei gleichzeitiger Steigerung der Raum-Zeit-Ausbeute - verringert wird.

Eine andere Möglichkeit des Hotspotmanagements auf Seiten des Reaktionsgases bei gekühlten Rohrbündelreaktoren wird beispielsweise in der WO 2018/019761 A1 und der WO 2018/019760 A1 beschrieben. Dort wird eine deutliche Reduktion des Hotspots auf der Prozessgasseite erreicht, indem auf Kühlmittelseite eine erhöhte Temperaturdifferenz durch gezielte Anpassung (in diesem Fall durch eine Verringerung) des Kühlmittelstromes zugelassen wird.

Die vorliegende Erfindung stellt sich die Aufgabe, verbesserte und effektivere Möglichkeiten zur Herstellung von Zielverbindungen der genannten Art aufzuzeigen, mittels derer insbesondere ein sicherer Betrieb ohne Produktionsausfall auch bei Störungen für einen wirtschaftlichen Anlagenbetrieb gewährleistet werden kann.

### Offenbarung der Erfindung

Die vorstehend genannte Aufgabe wird durch die in den jeweiligen unabhängigen Patentansprüchen angegeben Maßnahmen gelöst. Bevorzugte Ausgestaltungen sind jeweils Gegenstand der abhängigen Patentansprüche und der nachfolgenden Beschreibung.

In einer großtechnischen Anlage ist ein sicherer Betrieb ohne Produktionsausfall für den wirtschaftlichen Anlagenbetrieb von großer Bedeutung. Ein thermisch instabiler Betriebszustand des Reaktors kann jedoch herkömmlicherweise mindestens zu Teilabschaltungen einer Produktionsanlage und somit zu Produktionsausfällen führen.

Gemäß den weiter unten folgenden weiteren Beschreibungen des technischen Hintergrunds der Erfindung zeichnet sich ein thermisch instabiler Betriebszustand in einem Rohrbündelreaktor der erläuterten Art (mindestens) durch unterschiedliche Trends in den Temperaturverläufen an unterschiedlichen Punkten in Flussrichtung eines axialen Temperaturprofils entlang des Katalysatorbetts aus. Daher führt eine erfolgreiche Deeskalation eines thermisch instabilen Betriebszustandes mindestens zu (i) einer Vereinheitlichung der Temperaturtrends im Katalysatorbett eines oder mehrerer Reaktionsrohre und/oder (ii) einem Absenken der Temperaturen in einem Katalysatorbett eines oder mehrerer Reaktionsrohre, insbesondere der Temperaturen in der Näher von lokalen Temperaturmaxima (Hotspots) im Katalysatorbett. Der Begriff einer "Deeskalation" soll dabei hier Maßnahmen beschreiben, die einen Reaktor von einem instabilen oder instabil zu werden drohenden Betriebszustand in einen stabilen, handhabbaren Betriebszustand überführen. Alternativ wird auch der Begriff "Stabilisierung" verwendet.

Eine übliche Möglichkeit zur Deeskalation eines thermisch instabilen Betriebszustandes und insbesondere eines beginnenden thermischen Durchgehens ist eine Absenkung der Kühlmittel- (d.h. insbesondere Salzbad-)Temperatur, um auf diese Weise eine stärkere Kühlung des Reaktors zu erreichen. Allerdings zeichnen sich thermisch instabile Betriebszustände und insbesondere beginnendes thermisches Durchgehen durch kurze Zeitskalen von wenigen Minuten aus, wie auch an den weiter unten diskutierten Beispielen zu erkennen ist. Die Absenkung der Kühlmitteltemperatur hat aber insbesondere den entscheidenden Nachteil, dass diese aufgrund der sehr großen Kühlmittelmenge und entsprechenden Wärmekapazität insbesondere in einem großtechnischen Reaktor nur über deutlich längere Zeiträume erfolgen kann. Somit erfolgt eine Kühlmitteltemperaturabsenkung üblicherweise viel zu langsam und kann ein thermisch instabiles Verhalten und insbesondere ein thermisches Durchgehen nicht deeskalieren, d.h. das System nicht ausreichend stabilisieren.

Eine weitere Möglichkeit ist die Absenkung des Systemdrucks und damit Absenkung der Partialdrücke der Reaktanden, wodurch Reaktionsgeschwindigkeiten und somit die Wärmeproduktion deutlich verringert und dadurch auch ein thermisch instabiler Betriebszustand vermieden werden können. Die Absenkung des Drucks hat in einem kommerziellen Prozess allerdings zwei entscheidende Nachteile: (i) Auch diese Maßnahme kann in einer kommerziellen, großtechnischen Anlage nur langsam erfolgen und (ii) hat eine Druckabsenkung in einer kommerziellen Anlage direkten Einfluss auf die nachfolgenden Verfahrensschritte bzw. erfordert eine geeignete Anpassung dieser Verfahrensschritte.

Es sind also aus dem Stand der Technik zwar grundsätzlich Verfahren eines Hotspotmanagements, ggf. auch verbunden mit einer Effizienzsteigerung des

Verfahrens, bekannt, jedoch zeigen diese Verfahren keine Ansätze oder Lösungen für die Prozesssteuerungen auf, die insbesondere erforderlich sind, um auch einen sicheren Anlagenbetrieb im Falle einer Betriebsstörung, die zu einem thermisch instabilen Betriebszustand bzw. zu einem thermischen Durchgehen führen könnte, zu gewährleisten. Ferner wurde in diesem Zusammenhang bisher auch keine geeignete Diagnostik- oder Detektionsmethode aufgezeigt, mit der ein mögliches thermisch instabiles Verhalten bzw. ein sich ankündigendes thermisches Durchgehen des Reaktors oder Teilbereiche innerhalb eines Reaktors oder Reaktionsrohrs frühzeitig erkannt werden kann.

Mit der vorliegenden Erfindung wird demgegenüber (i) eine Detektionsmethode aufgezeigt, mit der ein thermisch instabiler Zustand bzw. ein beginnendes thermisches Durchgehen eines (groß-)technischen Reaktors oder Teilbereichen innerhalb eines (groß-)technischen Reaktors oder einzelner Reaktionsrohre eines technischen Reaktors erkannt werden kann und (ii) werden prozess- bzw. betriebstechnische Verfahrensweisen zur schrittweisen Deeskalation eines beginnenden thermischen Durchgehens aufgezeigt, mit deren Hilfe weiterhin ein sicherer Anlagenbetrieb gewährleistet und somit gegenüber bekannten Verfahren eine unterbrechungsfreie, stabile Produkterzeugung möglich ist.

Die vorliegende Erfindung schlägt dabei ein ganzheitliches Sicherheitskonzept für die Absicherung von Oxidationsreaktoren und insbesondere von ODH(E)-Reaktoren gegen thermisches Durchgehen bzw. thermisch instabilen Betrieb vor. Dabei umfasst die Erfindung sowohl eine Detektionsmethode zur Erkennung thermisch instabiler Zustände, als auch eine geeignete, insbesondere mehrstufige Deeskalationsmethode, die nach erfolgreicher Detektion ein sicheres Abfangen eines thermisch instabilen Zustands ermöglicht.

### Merkmale und Vorteile der Erfindung

Die Erfindung schlägt, zur Erreichung der genannten Ziele, ein Verfahren zur Herstellung einer Zielverbindung vor, bei dem ein wenigstens eine Eduktverbindung in Form eines oder mehrerer Kohlenwasserstoffe und insbesondere Sauerstoff enthaltendes Einsatzgemisch gebildet, auf parallel angeordnete Reaktionsrohre eines oder mehrerer Rohrbündelreaktoren verteilt, und in den Reaktionsrohren einer oxidativen katalytischen Umsetzung unter Verwendung eines oder mehrerer, in den Reaktionsrohren bereitgestellter Katalysatoren unterworfen wird.

Erfindungsgemäß werden Überwachungsmaßnahmen durchgeführt, die umfassen, auf Grundlage von Temperaturmessungen an mehreren Messstellen entlang eines oder mehrerer der Reaktionsrohre (und insbesondere in deren Innerem) jeweils zeitliche Temperaturverläufe zu ermitteln, auf Grundlage zumindest der Temperaturverläufe eine Stabilitätsbewertung der katalytischen Umsetzung vorzunehmen, und, wenn auf Grundlage der Stabilitätsbewertung ein (thermisch) instabiler Zustand der katalytischen Umsetzung als eingetreten oder wahrscheinlich eintretend bestimmt wird, geeignete Stabilisierungsmaßnahmen einzuleiten. Unter einem (thermisch) instabilen Zustand soll dabei insbesondere ein Zustand verstanden werden, der ohne eine Veränderung weiterer Betriebsparameter wie Kühlleistung oder Reaktandenmenge bzw. Reaktandenverdünnung eine unkontrolliert weiter ansteigende Temperatur zur Folge hat (thermisches Durchgehen, engl. Thermal Runaway).

Mit anderen Worten ist Gegenstand der vorliegenden Erfindung ein Verfahren zum frühzeitigen Erkennen und schrittweisen Deeskalation von thermisch instabilen Zuständen bei Verfahren zur (selektiven) Oxidation von Kohlenwasserstoffen (oder Gemischen von Kohlenwasserstoffen), wobei die Oxidation der Kohlenwasserstoffe in einem oder mehreren gekühlten, katalytischen Festbettreaktoren durchgeführt wird, wobei (i) der oder die die Reaktoren (bzw. entsprechend eine verfahrenstechnische Anlage) mit Messeinrichtungen versehen ist bzw. sind, mit denen die Temperatur entlang des Katalysatorbetts eines oder mehrerer Reaktionsrohre erfasst und aufgezeichnet werden kann, (ii) ein Algorithmus mit Hilfe der Temperaturdaten für dieses oder für jedes dieser Rohre beurteilt, ob ein thermisch instabiler Betriebszustand vorliegt, sowie in diesem Fall (iii) ein weiterer Algorithmus Deeskalationsmaßnahmen einleitet.

Die Temperaturmessungen an den mehreren Messstellen entlang des einen oder der mehreren Reaktionsrohre erfolgen insbesondere in Form einer Erfassung eines oder mehrerer in Flussrichtung axialer Temperaturprofile. In dem erfindungsgemäß vorgeschlagenen Verfahren können die Temperaturmessungen dabei eine Vielzahl von Temperaturmessungen in einem Reaktionsrohr oder in jedem von mehreren Reaktionsrohren umfassen, die unter Verwendung eines oder mehrerer Mehrpunkt-Temperaturfühler vorgenommen werden. Alternativ oder zusätzlich können die Temperaturmessungen auch eine Vielzahl von Temperaturmessungen in unterschiedlichen der Reaktionsrohre umfassen, die unter Verwendung mehrerer Einzelpunkt-Temperaturfühler vorgenommen werden. Zu entsprechenden Ausgestaltungen sei auf die obigen Erläuterungen zu Ein- und Mehrpunkt-Temperaturfühlern ausführlich verwiesen.

In dem erfindungsgemäßen Verfahren umfasst die Stabilitätsauswertung insbesondere die vergleichende Bewertung der Temperaturverläufe an zumindest zwei der Messstellen, die zur Beurteilung des in Flussrichtung axialen Temperaturprofils des bzw. der Katalysatorbetten herangezogen werden. Der instabile Zustand der katalytischen Umsetzung wird insbesondere dann als eingetreten oder wahrscheinlich eintretend erkannt, wenn an den zumindest zwei der Messtellen über mehr als einen vorgegebenen Zeitraum gegenläufige Temperaturtrends in mehr als einem vorgegebenen Umfang ermittelt werden. Der vorgegebene Zeitraum beträgt dabei insbesondere mehr als 0,5 Minuten, mehr als 1 Minute, mehr als 2 Minuten und bis zu 3 Minuten, und/oder der vorgegebene Umfang beträgt insbesondere mehr als 0,5%, mehr als 1%, mehr als 2%, mehr als 5% oder mehr als 10% im Vergleich zu einem Temperaturwert zu Beginn des vorgegebenen Zeitraums oder, alternativ, im Vergleich zu einem (gleitenden) Mittelwert.

Die erläuterten Maßnahmen können insbesondere die kontinuierliche Bestimmung und zeitliche Mittelung und somit Erfassung des Verlaufs jeder einzelnen Messtelle entlang eines Reaktionsrohrs bzw. entlang eines in Flussrichtung axialen Temperaturprofils umfassen. Auf dieser Basis kann eine kontinuierliche Bestimmung und Ermittlung des relativen Werteverlaufs (in Form von Trends) der einzelnen Messtellen untereinander erfolgen. Bei Erkennung eines gegenläufigen Trends von Temperaturmessstellen in Flussrichtung entlang eines Reaktionsrohrs bzw. entlang eines in Flussrichtung axialen Temperaturprofils kann insbesondere dann eine entsprechende Reaktion wie beispielsweise eine Alarmierung erfolgen, wenn in Flussrichtung der gegenläufige Trend in dem erwähnten vorgegebenen Zeitraum vorliegt. Ein gegenläufiger Trend in den Temperaturen eines in Flussrichtung axialen Temperaturprofils deutet im Sinne der Erfindung genau dann auf ein thermisch instabilen Betriebszustand hin, wenn im Reaktor bzw. im Katalysatorbett eines Reaktionsrohr in Flussrichtung entlang des Katalysatorbetts an mindestens einer Stelle im Katalysatorbett ein Wechsel in den Temperaturtrends der einzelnen Messstellen von ansteigend hin zu abfallend stattfindet. Der thermische Betriebszustand gilt im Sinne der Erfindung insbesondere dann als stabil, wenn die Temperaturtrends in einem Katalysatorbett eines Reaktionsrohrs bzw. in mehreren Reaktionsrohren oder im gesamten Reaktor in Flussrichtung alle die gleiche Richtung, d.h. alle ansteigend oder alle abfallend, sind oder im Rahmen von Messwertschwankungen bzw. Messfehlern konstant verlaufen.

In dem vorgeschlagenen Verfahren kann der instabile Zustand der katalytischen Umsetzung daher insbesondere dann als nicht eingetreten oder nicht wahrscheinlich eintretend bestimmt werden, wenn an den zumindest allen der Messtellen gleichläufige Temperaturtrends oder (im Wesentlichen) konstante Temperaturen ermittelt werden.

Die oben beschriebene Überwachung kann beispielsweise automatisch aktiviert werden, sobald ein bestimmter Zeitraum seit der letzten, vom Betriebspersonal vorgenommenen Änderung von relevanten Betriebssollwerten im Reaktorsystem verstrichen ist. Solch relevante Betriebssollwerte umfassen beispielsweise, aber nicht ausschließlich, eine Temperatur und/oder Umwälzmenge des Reaktorkühlmediums, eine Menge und/oder Zusammensetzung des Einsatzgemischs und/oder einen Betriebsdruck des einen oder der mehreren Reaktoren.

Somit kann in einer Ausgestaltung der Erfindung vorgesehen sein, dass die Überwachungsmaßnahmen erst dann durchgeführt werden, wenn ein vorgegebener Zeitraum von insbesondere mehr als 1 Minute, 10 Minuten oder 1 Stunde seit einer letzten vorgenommenen Änderung von eine Reaktionsstabilität beeinflussenden Betriebssollwerten verstrichen ist.

Die Ermittlung der Temperaturtrends im Verlauf einer Messstelle und somit auch die Erfassung von gegenläufigen Temperaturtrends kann in allen Ausgestaltungen der Erfindung mathematisch auf unterschiedliche Weise umgesetzt werden. Beispielsweise kann auf numerische Weise die Ableitung des Verlaufs jeder Messstelle gebildet werden. Eine positive Ableitung (d.h. der Wert der Ableitung ist größer als Null) einer Kurve bzw. Funktion, hier eines Temperaturverlaufs, bedeutet einen steigenden Trend in der Temperaturkurve, d.h. steigende Temperatur. Entsprechend bedeutet eine negative Ableitung (d.h. der Wert der Ableitung ist kleiner als Null) eine sinkende Temperatur. Entsprechend liegt ein gegenläufiger Trend zweier oder mehrerer Temperaturkurven vor, wenn deren Ableitungen unterschiedliche Vorzeichen aufweisen, d.h. einerseits größer und andererseits kleiner als Null sind.

Um eventuelle Fehlalarme bzw. Fehlreaktionen zu minimieren, kann es vorteilhaft sein, die Trendbestimmung mit einem mathematischen Filter zur Kurvenglättung zu versehen. Auf diese Weise können Schwankungen in den Messwerten und den zugehörigen Ableitungen teilweise ausgeglichen werden. Es versteht sich, dass die Ermittlung der Temperaturtrends auch mit anderen geeigneten mathematischen Methoden erfolgen kann und die hier aufgeführten lediglich als Beispiel dienen.

In Ergänzung zum vorstehend Genannten können die Überwachungsmaßnahmen in einer Ausgestaltung der Erfindung auch umfassen, auf Grundlage einer Sauerstoffmessung an einem stromabwärtigen Ende und/oder stromab eines oder mehrerer der Reaktionsrohre ein oder mehrere Sauerstoffgehalte eines oder mehrerer Prozessgasströme zu ermitteln, wobei die Stabilitätsbewertung unter Berücksichtigung des oder der Sauerstoffgehalte durchgeführt wird.

Hierbei kann ein ein vergleichbares Vorgehen wie bei der Überwachung der Temperaturen vorteilhaft sein, allerdings mit dem vereinfachenden Umstand, dass hier nur der Trend einer einzelnen Kurve ermittelt werden muss. Beispielsweise kann ein gleitender Mittelwert über die letzten bis zu 10 Minuten, 30 Minuten, 1 Stunde oder 2 Stunden kontinuierlich ermittelt werden, und im Rahmen der Überwachung kann geprüft werden, ob es in einem Zeitraum von mindestens 0,5 Minuten, mindestens 1 Minute, mindestens 2 Minuten und bis zu 5 Minuten zu einem dauerhaften Abfall von mindestens 10%, 20% oder 50% gegenüber dem gleitenden Mittelwert kommt. Dies deutet im Sinne der Erfindung auf einen thermisch instabilen Betrieb hin. Entsprechend liegt im Sinne der Erfindung ein stabiler thermischer Zustand vor, wenn der Trend der Kurve der Sauerstoffkonzentration sich konstant in einem engen Bereich um den Sollwert bzw. mittleren Messwertes bewegt oder aber ansteigend verläuft.

Die vorliegende Erfindung beruht insbesondere auf der Erkenntnis, dass sich bei allen beobachteten thermisch instabilen Betriebszuständen bzw. beginnendem oder vollständigem thermischem Durchgehen sich stets zwei wiederkehrende Charakteristika zeigen, die auch anhand derunten angegebenen Beispiele aufgeführt sind und die im Rahmen eines online-Monitoring von Prozessdaten kontinuierlich erfasst werden können. Diese sind (i) gegenläufige Trends bzw. ein Auseinanderdriften der Temperaturen im Katalysatorbett stromauf bis einschließlich der thermisch instabilen Zone (Hotspot) und stromab der thermisch instabilen Zone und (ii) ein vergleichsweise plötzlicher Abfall der Sauerstoffkonzentration im Prozessgas stromab des Reaktors. In diesem Zusammenhang sei auch erwähnt, dass eine gewisse Mindestkonzentration an Sauerstoff im Prozessgas stromab des Reaktors zum Erhalt Katalysatorstabilität notwendig ist. Eine zu lange Zeit unter sauerstofffreien, reduktiven Bedingungen bei (hoher, prozesstypischer) Temperatur führt zur Schädigung des speziell in der ODH(E) eingesetzten Katalysators des oben genannten Typs.

Sämtliche oder ein Teil der erfindungsgemäß vorgeschlagenen Maßnahmen können in Form eines Algorithmus bzw. von Algorithmen in einer Prozesssteuerung hinterlegt werden. Somit kann die Überwachung des Betriebs und das frühzeitige Erkennen von thermisch instabilen Betriebszuständen weitgehend automatisiert erfolgen, was die gesamte Betriebssicherheit deutlich erhöht, da das Erkennen eines instabilen Betriebs so vom menschlichen Faktor unabhängig wird.

Im Gegensatz zu dem in den untenstehenden Beispielen verwendeten Pilotreaktor bestehen kommerzielle Reaktoren aus einer großen Anzahl an Reaktionsrohren (mehrere 1.000 bis mehrere 10.000). Die Reaktionsführung in den verschiedenen Reaktionsrohren wird zwangsläufig einer Schwankungsbreite unterworfen sein, z.B. aufgrund von unterschiedlichen Strömungsbedingungen auf der Kühlmittelseite oder auch variierenden Mischungsbedingungen auf der Prozessseite. Eine instrumentenbasierte Überwachung jedes einzelnen Reaktionsrohrs ist praktisch nicht möglich. Insbesondere kann die Prozessgaszusammensetzung am Reaktoraustritt typischerweise nur für das resultierende Gesamtgemisch gemessen werden. Wie oben beschrieben, kann eine erhöhte Schwankungsbreite dieser Messgröße, z.B. des Sauerstoffs am Reaktoraustritt, nur auf einen global instabilen Reaktorbetrieb hindeuten, da bei der Überwachung des Prozessgases zwangsläufig eine Mittelung über alle Reaktionsrohre entsteht, und somit erst zu einem fortgeschrittenen Eskalationsstadium in die Prozesssteuerung einfließen.

Besonders geeignet für die frühzeitige Detektion von instabilen Reaktionsbedingungen in einzelnen Reaktorbereichen ist daher die Analyse von Temperaturmessungen in einzelnen Reaktionsrohren, wie oben beschrieben. In der industriellen Praxis ist es gebräuchlich, eine begrenzte Anzahl von Reaktionsrohren mit Temperaturfühlern entlang des Katalysatorbetts auszustatten. Mit diesen einzelnen Messstellen können repräsentative Stichproben der Temperaturbedingungen in unterschiedlichen Bereichen des Reaktors realisiert werden. Dementsprechend sieht die Erfindung vor, die oben am Beispiel eines einzelnen Reaktionsrohrs erläuterte Detektionsmethode instabiler Reaktorbetriebszustände jeweils für mindestens einen Teil, bevorzugt jedoch für alle mit Temperaturmessstellen ausgestatteten Reaktionsrohre eines großtechnischen Reaktors anzuwenden. Somit können durch die Erfindung auch lokal auftretende thermisch instabile Betriebsbedingungen frühzeitig detektiert werden. Die globale Sauerstoffüberwachung am Austritt des Reaktors liefert zudem die Möglichkeit, den lokalen Messwerten eine für den globalen Reaktorbetriebszustand relevante Messgröße beiseite zu stellen.

Nachdem zuvor eine Methode zur frühzeitigen und automatisierten Erkennen von thermisch instabilen Betriebszuständen aufgezeigt wurde, soll nun ein Verfahren zur stufenweisen Deeskalation von thermisch instabilen Betriebszuständen und/oder beginnenden thermischen Durchgehen näher erläutert werden.

Zusammengefasst können die erfindungsgemäß eingeleiteten Stabilisierungsmaßnahmen Maßnahmen unterschiedlicher Eingriffsschwere umfassen, wobei eine Maßnahme einer größeren Eingriffsschwere dann ergriffen wird, wenn eine Maßnahme geringerer Eingriffsschwere nach deren Durchführung als nicht ausreichend stabilisierend bestimmt und/oder als voraussichtlich nicht ausreichend stabilisierend bestimmt wird.

Die Maßnahmen unterschiedlicher Eingriffsschwere können dabei insbesondere die folgenden, in ihrer Eingriffsschwere von (a) bis (c) zunehmenden Maßnahmen oder Kombinationen hiervon umfassen: (a) eine schrittweise Erhöhung der stündlichen Raumgeschwindigkeit des Einsatzgemischs (engl. Gas Hourly Space Velocity, GHSV) durch eine ausschließliche Erhöhung einer zeitlichen Menge der wenigstens einen Eduktverbindung, d.h. des einen oder der mehreren Kohlenwasserstoffe, (b) eine schrittweise Erhöhung der stündlichen Raumgeschwindigkeit des Einsatzgemischs durch eine Erhöhung der zeitlichen Menge wenigstens einer in dem Einsatzgemisch enthaltenen Inertkomponente, und (c) ein Unterbinden der Reaktandenzufuhr und Inertisierung des einen oder der mehreren Rohrbündelreaktoren.

Die ersten beiden Stufen (a) und (b) können jeweils noch in verschiedene Abstufungen unterteilt werden und daher insbesondere auch so durchgeführt werden, dass eine weitere Übergabe des Prozessgases in den Trennteil einer großtechnischen Anlage weiterhin gewährleistet ist, d.h. eine kontinuierliche Produktion kann weiterhin spezifikationsgerecht aufrechterhalten werden. Insbesondere können die Maßnahmen der Stufen (a) und (b) auch kombiniert werden, um eine besonders intensive Deeskalationsstufe zu erhalten und dennoch den Produktionsbetrieb aufrecht erhalten zu können. Bei der dritten Stufe (c), also dem Stopp der Reaktandenzufuhr, ist dies nicht mehr möglich.

Insgesamt handelt es sich bei der Erfindung bei Einsatz der erwähnten Deeskalationsmaßnahmen um ein Verfahren zum frühzeitigen Erkennen eines thermisch instabilen Betriebszustandes bzw. eines beginnenden thermischen Durchgehens und zum Ergreifen geeigneter Gegenmaßnahmen, wobei die Intensität der Gegenmaßnahmen je nach Intensität der thermischen Instabilität gesteigert werden kann, so dass zunächst der Produktionsbetrieb der Anlage zumindest in einem gewissen Umfang aufrechterhalten werden kann. Erst ein unabwendbares thermisches Durchgehen würde die Sicherheitsabschaltung des Reaktors (und damit Stopp des Produktionsbetriebs) bedeuten.

Die oxidative katalytische Umsetzung wird insbesondere unter Verwendung eines oder mehrerer, die Metalle Molybdän, Vanadium, Niob und optional Tellur enthaltender Katalysatoren durchgeführt, der oder die in Reaktionszonen des einen oder der mehreren Rohrbündelreaktoren bereitgestellt werden, die in einer Strömungsrichtung hintereinander angeordnet sind und in der Strömungsrichtung durchströmt werden. Der eine oder oder die mehreren Katalysatoren kann bzw. können in den mehreren Reaktionszonen mit einer unterschiedlichen Katalysatorbeladung und/oder einer unterschiedlichen Katalysatoraktivität pro Raumeinheit bereitgestellt sein, wodurch eine Aktivitätsabstufung zwischen den Reaktionszonen erzielt wird.

Wie erwähnt, eignet sich die Erfindung insbesondere für die Verwendung im Zusammenhang mit der ODH, insbesondere der ODHE, so dass insbesondere Ethan als Einsatzverbindung verwendet wird und das oxidative katalytische Verfahren als oxidative Dehydrierung des Ethans durchgeführt wird. Auch eine ODH höherer Kohlenwasserstoffe, beispielsweise von Propan, Butan, Pentan, und/oder Hexan oder analoger Verbindungen kann durchgeführt werden. Ebenso ist die Erfindung für andere oxidative katalytische Umsetzungen geeignet, wie beispielsweise der Oxidation von Benzol zu Maleinsäureanhydrid oder von Xylol zu Phthalsäureanhydrid.

Die vorliegende Erfindung sieht insbesondere vor, das oxidative katalytische Verfahren bei einer Temperatur des oder der Katalysatoren in einem Bereich zwischen 240 und 500 °C, insbesondere zwischen 280 und 450 °C, weiter insbesondere zwischen 300 und 400 °C durchzuführen, und/oder dieses mit einem Gesamtdruck des Einsatzgemischs an einem Eintritt des oder der Rohrbündelreaktoren von 1 bis 10 bar (abs.), insbesondere 2 bis 6 bar (abs.) durchzuführen.

In dem erfindungsgemäßen Verfahren werden die Reaktionsrohre insbesondere unter Verwendung eines oder mehrerer, die Reaktionsrohre umfließender Kühlmedien gekühlt, wie bereits zuvor erwähnt.

In Ausgestaltungen der Erfindung können unterschiedliche Abschnitte der Reaktionsrohre unter Verwendung unterschiedlicher Kühlmedien, unter Verwendung desselben Kühlmediums in unterschiedlichen Kühlmedienkreisläufen, und/oder unter Verwendung desselben oder unterschiedlicher Kühlmedien in unterschiedlichen oder gleichen Strömungsrichtungen gekühlt werden.

In dem erfindungsgemäßen Verfahren kann das Einsatzgemisch einen Wasseranteil enthalten, der zwischen 5 und 95 Vol.-%, insbesondere 10 und 50 Vol.-%, insbesondere 14 und 35 Vol.-% eingestellt wird. Das molare Verhältnis von Wasser zu Ethan im Reaktionseinsatzstrom kann insbesondere mindestens 0,23 betragen.

Eine Anlage zur Herstellung einer Zielverbindung mit einem oder mehreren, parallel angeordnete Reaktionsrohre aufweisenden Rohrbündelreaktoren, die dafür eingerichtet ist, ein wenigstens eine Eduktverbindung in Form eines oder mehrerer Kohlenwasserstoffe enthaltendes Einsatzgemisch zu bilden, auf Reaktionsrohre des oder der Rohrbündelreaktoren zu verteilen, und in den Reaktionsrohren einer oxidativen katalytischen Umsetzung zu unterwerfen, ist ebenfalls Gegenstand der vorliegenden Erfindung.

Diese ist dafür eingerichtet, Überwachungsmaßnahmen durchzuführen, die umfassen, auf Grundlage von Temperaturmessungen an mehreren Messstellen entlang eines oder mehrerer der Reaktionsrohre bzw entlang eines oder mehrerer axialer Temperaturprofile jeweils zeitliche Temperaturverläufe zu ermitteln, auf Grundlage zumindest der Temperaturverläufe eine Stabilitätsbewertung der katalytischen Umsetzung vorzunehmen, und, wenn auf Grundlage der Stabilitätsbewertung ein instabiler Zustand der katalytischen Umsetzung als eingetreten oder wahrscheinlich eintretend bestimmt wird, Stabilisierungsmaßnahmen einzuleiten.

Zu Merkmalen und Vorteilen der erfindungsgemäß vorgeschlagenen Anlage und vorteilhafter Ausgestaltungen hiervon sei auf die obigen Ausführungen bezüglich des erfindungsgemäßen Verfahrens und vorteilhafter Ausgestaltungen hiervon ausdrücklich verwiesen. Insbesondere ist die erfindungsgemäß vorgeschlagene Anlage bzw. sind entsprechende Ausgestaltungen dazu eingerichtet, entsprechende Verfahrensvarianten durchzuführen.

### Ausführungsbeispiele

Die Erfindung wird nachfolgend anhand von Beispielen, die Ausgestaltungen der Erfindung entsprechen, und nicht erfindungsgemäßen Vergleichsbeispielen sowie zugehörigen Figuren und Tabellen weiter erläutert.
Figur 1 veranschaulicht ein Verfahren gemäß einer Ausgestaltung der Erfindung in Form eines schematischen Ablaufplans.
Figur 2 veranschaulicht Parameter im zeitlichen Verlauf an unterschiedlichen Positionen eines Rohrbündelreaktors gemäß einem zur Illustration der Erfindung verwendeten Beispiels.
Figur 3 veranschaulicht Parameter im zeitlichen Verlauf an unterschiedlichen Positionen eines Rohrbündelreaktors gemäß einem zur Illustration der Erfindung verwendeten weiteren Beispiel.
Figur 4 veranschaulicht Parameter im zeitlichen Verlauf an unterschiedlichen Positionen eines Rohrbündelreaktors gemäß einem zur Illustration der vorliegenden Erfindung verwendeten weiteren Beispiel.
Figur 5 veranschaulicht einen Rohrbündelreaktor gemäß einer Ausgestaltung der vorliegenden Erfindung in vereinfachter schematischer Darstellung.

Wird nachfolgend auf Aspekte von erfindungsgemäß oder gemäß Ausführungsformen der Erfindung ausgestalteten Verfahren Bezug genommen, betreffen diese entsprechende Anlagen und ihre Ausgestaltungen in gleicher Weise.

Insgesamt schlägt die vorliegende Erfindung, wie bereits zuvor erläutert, ein Verfahren zum frühzeitigen Erkennen eines thermisch instabilen Betriebszustandes bzw. eines beginnenden thermischen Durchgehens und zum Ergreifen geeigneter Gegenmaßnahmen, wobei die Intensität der Gegenmaßnahmen je nach Intensität der thermischen Instabilität gesteigert werden kann, sodass zunächst der Produktionsbetrieb der Anlage aufrechterhalten werden kann. Erst ein unabwendbares thermisches Durchgehen würde die Sicherheitsabschaltung des Reaktors (und damit Stopp des Produktionsbetriebs) bedeuten.

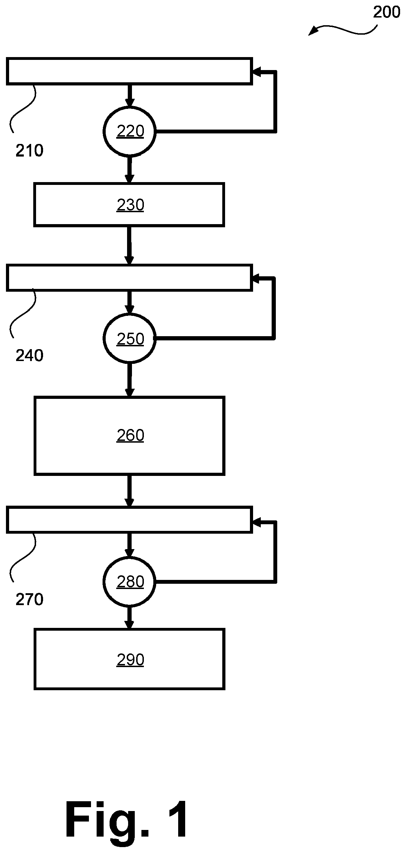

Dieses Verfahren lässt sich in Ausgestaltungen der Erfindung steuerungstechnisch weitgehend oder vollständig automatisieren, indem beispielsweise ein entsprechender Algorithmus in ein Prozessleitsystem eingepflegt wird. Auf diese Weise kann ein Verfahren gemäß einer in Figur 1 veranschaulichten Ausgestaltung implementiert werden, welches insgesamt mit 200 bezeichnet ist.

In einem Schritt 210 kann dabei eine Anwendung des oben bereits angesprcohenen beschriebenen Algorithmus zur Erkennung eines thermisch instabilen Betriebszustandes erfolgen. Allgemeiner gesprochen können in dem Schritt 210 Überwachungsmaßnahmen durchgeführt werden, die umfassen, auf Grundlage von Temperaturmessungen an mehreren Messstellen entlang eines oder mehrerer der Reaktionsrohre bzw. entlang eines oder mehrerer axialer Temperaturprofile eines oder mehrerer Reaktoren während einer mehrfach erläuterten katalytischen Umsetzung jeweils zeitliche Temperaturverläufe zu ermitteln, und auf Grundlage zumindest der Temperaturverläufe eine Stabilitätsbewertung der katalytischen Umsetzung vorzunehmen.

Liegt ein thermisch instabiler Betrieb vor, wird eine entsprechende Deeskalierungskaskade in Gang gesetzt, wie hier beginnend mit dem Entscheidungsblock 220 veranschaulicht. Mit anderen Worten ist vorgesehen, dass, wenn auf Grundlage der Stabilitätsbewertung in Schritt 210 ein instabiler Zustand der katalytischen Umsetzung als eingetreten oder wahrscheinlich eintretend bestimmt wird, Stabilisierungsmaßnahmen eingeleitet werden.

Entsprechende Maßnahmen sind mit den Schritten 230, 260 und 290 veranschaulicht, wobei zwischen diesen jeweils mit dem Schritt 210 und dem Entscheidungsblock 220 vergleichbare Schritte 240 und 270 und Entscheidungsblöcke 250 und 280 vorgesehen sind. Es werden also in den Schritten 240 und 270 jeweils vergleichbar zu den zuvor erläuterten Überwachungsmaßnahmen ausgestaltete Überwachungsmaßnahmen durchgeführt und gemäß den Schritten 250 und 280 jeweils eine Stabilitätsbewertung der katalytischen Umsetzung vorgenommen. Nur dann, wenn aufgrund der zuvor vorgenommenen Stabilisierungsmaßnahme 230 bzw. 260 kein ausreichend stabiler Zustand erzielt werde kann, schreitet das Verfahren zur nächsten Stabilisierungsmaßnahme 260 bzw. 290 fort.

Gemäß Schritt 230 erfolgt dabei zunächst eine schrittweise Erhöhung der stündlichen Raumgeschwindigkeit des Einsatzgemischs durch eine ausschließliche Erhöhung einer zeitlichen Menge der wenigstens einen Eduktverbindung in Form des einen oder der mehreren Kohlenwasserstoffe (insbesondere aber nicht von Sauerstoff). Liegt weiterhin ein thermisch instabiler Zustand vor, kann ggf. die Intensität der Maßnahme dieser Stufe gesteigert werden (durch eine weitere Erhöhung des Kohlenwasserstoffstromes), oder es kann eine Maßnahme gemäß Schritt 260 angewandt. Andernfalls, d.h. wenn kein thermisch instabiler Zustand mehr vorliegt, also der thermisch instabile Zustand mit der Deeskalationsmaßnahme gemäß Schritt 230 behoben werden konnte, wird der neue Betriebszustand bis zur Beseitigung der den thermisch instabilen Betrieb verursachenden Betriebsstörung aufrechterhalten.

Eine Steigerung der Stabilisierungsmaßnahmen erfolgt gemäß Schritt 260, in dem eine schrittweise Erhöhung der stündlichen Raumgeschwindigkeit des Einsatzgemischs durch eine Erhöhung der zeitlichen Menge wenigstens einer in dem Einsatzgemisch enthaltenen Inertkomponente vorgenommen wird. Wie erwähnt, können dabei die Maßnahmen gemäß den Schritten 230 und 260 auch kombiniert werden. Falls nach Schritt 260 kein thermisch instabiler Betriebszustand mehr vorliegt, also der thermisch instabile Betriebszustand mit der vorhergehenden Deeskalationsmaßnahme gemäß Schritt 230 und/oder Schritt 260 behoben werden konnte, wird der neue, nun wieder stabile Betriebszustand bis zur Beseitigung der den thermisch instabilen Betrieb verursachenden Betriebsstörung aufrechterhalten.

Liegt nach Anwendung der gesteigerten Deeskalationsmaßnahmen gemäß den Schritten 230 und/oder 260 weiterhin ein thermisch instabiler Betriebszustand vor, ist von einem thermischen Durchgehen auszugehen. In diesem Fall wird Schritt 290 durchgeführt. Es kann dabei insbesondere eine Unterbindung der Reaktandenzufuhr und Inertisierung des einen oder der mehreren Rohrbündelreaktoren erfolgen. Mit anderen Worten kann der Reaktor bzw. können die Reaktoren über die Deeskalation des Schritts 290 abgestellt werden.

Nachfolgend sollen die gemäß entsprechender Ausgestaltungen der Erfindung vorgesehenenen Stabilisierungsmaßnahmen, deren technischer Hintergrund und deren jeweilige Wirkungen erläutert werden.

Eine schrittweise Erhöhung der stündlichen Raumgeschwindigkeit des Einsatzgemischs durch eine ausschließliche Erhöhung einer zeitlichen Menge der wenigstens einen Eduktverbindung wurde dabei zuvor mit (a) referenziert. Sie entspricht auch Schritt 230 gemäß Figur 1.

Im Rahmen der Erfindung hat sich überraschenderweise gezeigt, dass eine Deeskalierung eines thermisch instabilen Betriebszustandes bei unveränderter Kühlmitteltemperatur durch Erhöhung des (Massen- bzw. Volumen-) Stromes des zu oxidierenden Kohlenwasserstoffs, im Falle der ODHE also von Ethan, bei sonst unveränderten Bedingungen hinsichtlich der Ströme der anderen Komponenten im Reaktionseinsatzstrom oder hinsichtlich des Systemdrucks und insbesondere bei unveränderter Kühlmitteltemperatur erreicht werden kann. Die Erhöhung des Flusses des Kohlenwasserstoffs kann selbst in einer großtechnischen Anlage sehr rasch, d.h. in gleichen Zeitskalen wie das Auftreten von thermisch instabilen Betriebszuständen, erfolgen, sodass ein rasches Reagieren und somit Deeskalieren auf einen erkannten thermisch instabilen Betriebszustand möglich ist.

Die Erhöhung des Flusses des zu oxidierenden Kohlenwasserstoffs führt demnach, wie erfindungsgemäß erkannt wurde, mindestens zu einer Vereinheitlichung der Temperaturtrends im Katalysatorbett eines oder mehrerer Reaktionsrohre und, je nach Ausmaß der Flusssteigerung, zu einer Absenkung aller Temperaturen, aber insbesondere der Temperatur(en) in der Nähe des/der thermisch instabilen Bereiche in einem oder mehreren Reaktionsrohren.

Je nach Betriebsbedingungen des eigentlichen, stabilen Betriebspunkts, insbesondere je nach dem des für den normalen Betrieb eingestellten Wasser- (bzw. Dampf-) Ethan-Verhältnisses im Reaktionseinsatzstrom kann die Deeskalation des thermisch instabilen Betriebs auch zusätzlich dadurch verstärkt werden, dass durch die Erhöhung des Kohlenwasserstoffstromes der für die Aufrechterhaltung der Katalysatoraktivität notwendige Mindestwasseranteil im Reaktionseinsatzstrom (vgl. WO 2018/115418 A1) bzw. das minimal benötigte Wasser- (bzw. Dampf-) Ethan-Verhältnis von ca. 0,23 mol/mol unterschritten wird, was zusätzlich zu einer Absenkung der Katalysatoraktivität und somit Verstärkung des deeskalierenden Effekts führt.

Bei dieser Art der Deeskalation kann auch mindestens ein Stopp im Absinken der Sauerstoffkonzentration im Prozessgas stromab des Katalysatorbetts - und somit auch im dem Teil des Katalysatorbetts, das sich stromab der thermisch instabilen Zone befindet, erreicht werden, wie im unten erläuterten Beispiel 3 (und zugehöriger Tabelle 1 und Figur 4) demonstriert ist. Je nach Höhe der Flusssteigerung des zu oxidierenden Kohlenwasserstoffs wird die Sauerstoffkonzentration sogar leicht angehoben. Diese Erhöhung der Sauerstoffkonzentration im Prozessgas stromab des Reaktors ist dabei moderat und kann insbesondere niedrig genug gehalten werden, dass eine weitere Übergabe des Prozessgases in den Trennteil insbesondere einer (großtechnischen) ODHE-Anlage erfolgen kann.

Insbesondere bei einer großtechnischen ODHE-Anlage ist die Restsauerstoffkonzentration im (trockenen) Prozessgas stromab des Reaktors ein wichtiges Kriterium, ob das Prozessgas weiter in den Trennteil der Anlage übergeben werden darf, da es infolge der Trennoperationen zu einer Anreicherung von Sauerstoff im Prozessgas bis hin zur Bildung von zündfähigen Gemischen im kryogenen Teil der Trennsequenz kommen kann. Aufgrund von Sicherheitsbetrachtungen zur Vermeidung von explosionsfähigen Gemischen in einem nachfolgenden Trenn- und Aufreinigungsteil einer ODHE-Anlage liegt dieser Grenzwert für die Restsauerstoffkonzentration im trockenen Prozessgas, d.h. im Prozessgas stromab der Kondensatabtrennung stromab des ODHE-Reaktors, üblicherweise bei weniger als 2,5 mol%, bevorzugt weniger als 1,5 mol%, besonders bevorzugt weniger als 1 mol%.

Die vorangegangene beschriebene alleinige Erhöhung des Stromes des zu oxidierenden Kohlenwasserstoffs entspricht zugleich auch einer Erhöhung der GHSV, oder, anders ausgedrückt, einer Verkürzung der Verweilzeit, im Reaktor.

In einer nicht erfindungsgemäßen Ausgestaltung kann die GHSV auch dadurch erhöht werden, dass das Verhältnis von Oxidationsmittel zum Kohlenwasserstoff im Reaktionseinsatz gleich behalten wird. In diesem Fall führt eine Erhöhung des Kohlenwasserstoffstromes auch gleichzeitig zu einer entsprechenden Erhöhung des Oxidationsmittelstromes, im Falle der ODH-E also Sauerstoff. Wie im unten aufgeführten Beispiel 3 (und in Figur 4) demonstriert, führt dies zwar zu einer Absenkung der Temperaturen in einem Teil des Katalysatorbetts, auch in den Regionen der lokalen Hotspots. Jedoch kommt es auch in einem in Flussrichtung stromab befindlichen Teils des Katalysatorbetts, insbesondere in dem Teil, der bereits im Normalbetrieb aufgrund des über die Bettlänge fortgeschrittenen Umsatzes eine gewisse relative Sauerstoffarmut aufweist, aber insbesondere im Falle einer stromauf befindlichen thermisch instabilen Zone eine starke Sauerstoffarmut aufweist, zu einem deutlichen Anstieg der Temperatur. Dieser Temperaturanstieg ist darauf zurückzuführen, dass in diesem Bereich nun aufgrund des insgesamt durch die gesteigerte GHSV niedrigeren Umsatzes verglichen mit dem Normalbetrieb deutlich mehr Sauerstoff, also ein höherer Sauerstoffpartialdruck, vorliegt, was in diesem Bereich des Katalysatorbetts zu einer erhöhten Reaktionsgeschwindigkeit und damit einhergehend zu einer Temperaturerhöhung führt.

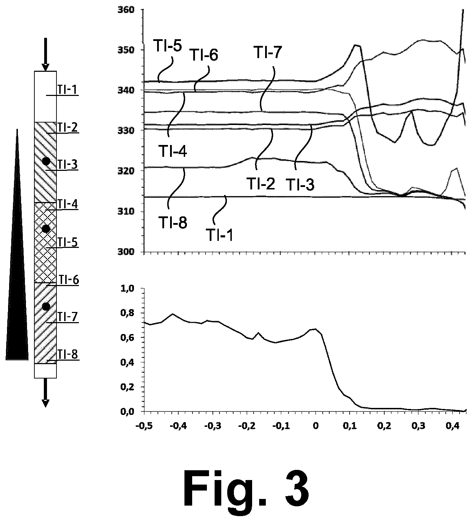

Bei Vorliegen eines echt thermisch instabilen Verhaltens bzw. eines beginnenden thermischen Durchgehens, ist das Reaktionsgas im Katalysatorbett stromab der thermisch instabilen Zone stark an Sauerstoff verarmt, im Extremfall sogar sauerstofffrei (vgl. auch Erklärungen zu Beispiel 2 und Figur 3 unten). Gemäß den Ausführungen zum technischen Hintergrund ebendort kommt es dann zur Akkumulation von reaktiven (Zwischen-) Produkten in dieser sauerstoffarmen Zone. Wenn es aber dann aufgrund einer Erhöhung des Sauerstoff- und Kohlenwasserstoffflusses zu einer Erhöhung der Sauerstoffkonzentration bzw. des Sauerstoffpartialdrucks im an Sauerstoff verarmten Teil des Katalysatorbetts stromab der thermischen Instabilität kommt, führt dies zu einer plötzlichen Abreaktion der akkumulierten Spezies und somit sehr wahrscheinlich zu einem nicht mehr aufzuhaltenden thermischen Durchgehen im Katalysatorbett stromab der eigentlichen thermischen Instabilität. Das bedeutet, dass diese Art der GHSV-Erhöhung durch Beibehaltung des Oxidationsmittel-Kohlenwasserstoffverhältnisses, im Falle der ODHE des Sauerstoff-Ethan-Verhältnisses, keine praktikable Deeskalationsmethode darstellt. Zudem führt diese Methode auch zu einem zu großen Anstieg der Restsauerstoffkonzentration im Prozessgas stromab des Reaktors, so dass eine Übergabe in den Trennteil einer ODHE-Anlage nicht mehr erfolgen dürfte. Somit wäre die Produktion unterbrochen.

Eine schrittweise Erhöhung der stündlichen Raumgeschwindigkeit des Einsatzgemischs durch eine Erhöhung der zeitlichen Menge wenigstens einer in dem Einsatzgemisch enthaltenen Inertkomponente wurde zuvor mit (b) referenziert. Sie entspricht auch Schritt 260 gemäß Figur 1.

Diese weist einen ähnlichen Effekt auf die Temperaturen auf die Temperaturen im Katalysatorbett stromab einer thermischen Instabilität auf, wenngleich diese deutlich schwächer als bei der gleichzeitigen Erhöhung des Oxidationsmittels und des Kohlenwasserstoffs ist. Die Erhöhung oder Zudosierung einer Inertgaskomponente im Reaktionsgas (vgl. unten aufgeführtes Beispiel und Figur 3).

Aufgrund des stark verdünnenden Effekts, d.h. deutlichen Absenkung der Partialdrücke der Reaktanden, zusätzlich zur verstärkten Wärmeabfuhr aufgrund des erhöhten Gesamtflusses, ließe sich der Temperaturanstieg im Katalysatorbett stromab der thermischen Instabilität bei der Zudosierung von einem Inertgas begrenzen, so dass dort, im Gegensatz zur GHSV-Erhöhung durch gleichzeitige Erhöhung von Oxidationsmittel und Kohlenwasserstoff, ein thermisches Durchgehen vermieden werden kann. Daher führt diese Methode bei der Erhöhung der GHSV um ähnliche Werte wie bei der Erhöhung der GHSV allein durch Erhöhung des Kohlenwasserstoffstroms zu deutlich größeren Anstiegen der Sauerstoffkonzentration im (trockenen) Prozessgas stromab des Reaktors. Somit handelt es sich im Rahmen der Erfindung auch hiermit um eine anwendbare Methode zur Deeskalation eines thermisch instabilen Betriebszustandes, vor allem wenn diese Maßnahme zusätzlich zu der bereits bei Deeskalationsstufe (a) bzw. Schritt 230 ergriffenen Maßnahmen angewandt wird.

Durch den verstärkenden Effekt der Partialdruckabsenkung der Reaktanden durch Inertdosierung ist diese Methode sogar sensitiver als die reine Erhöhung des Kohlenwasserstoffstromes und wäre somit als eine weitere Deeskalationsstufe anwendbar, wenn der thermisch instabile Betrieb durch Erhöhung des Kohlenwasserstoffstromes nicht mehr abwendbar ist. Inertgaskompontenten können dabei Edelgase, Stickstoff, Methan oder Wasserdampf sein. Im Falle der ODHE ist die Erhöhung des Wasserdampfstromes des Reaktionseinsatzstromes als Inertkomponente gegenüber der Zudosierung eines anderen Inertgases von besonderem Vorteil, da Wasserdampf bereits ein Teil des Reaktionseinsatzstromes bei der ODHE ist. Zusätzlich kann natürlich eine weitere Inertgaskomponente hinzudosiert werden. Bei der richtigen Einstellung der GHSV über die Inertmenge, ließe sich also auch im Falle der ODHE der Produktionsbetrieb aufrechterhalten, solange die Sauerstoffkonzentration im trockenen Prozessgas stromab des Reaktors einen Maximalwert von ca. 2 mol% nicht überschreitet.

Das Unterbinden der Reaktandenzufuhr und die Inertisierung des einen oder der mehreren Rohrbündelreaktoren wurde zuvor mit (c) referenziert. Sie entspricht auch Schritt 290 gemäß Figur 1.

Es handelt sich hierbei um die ultimative Methode zu Verhinderung eines thermischen Durchgehens. Es sei jedoch hier darauf hingewiesen, dass dies zum Stillstand der Anlage und somit zum Produktionsstopp führt. Für die Inertisierung kommen hier Gase aus der Stoffgruppe der Edelgase, Methan oder bevorzugt Stickstoff in Frage. Nachdem die Inertisierung solange erfolgt ist, bis die Temperaturen im Katalysatorbett etwa Salzbadniveau erreicht haben, sollte dem Inertgasstrom Sauerstoff zudosiert werden, wobei die Sauerstoffkonzentration im resultierenden Gasstrom in Schritten von ca. 0,5 mol% bis auf ca. 2 mol% erhöht wird, um eventuelle Akkumulationen im Katalysatorbett stromab der thermischen Instabilität oxidativ zu entfernen und das Katalysatorbett in diesem Bereich zu regenerieren. Eine langsame und schrittweise Erhöhung der Sauerstoffkonzentration im Eingangsstrom ist dabei wichtig, damit es bei dieser Regenerierung nicht zu einem thermischen Durchgehen kommt.

Wie zuvor erläutert, lässt sich die Sauerstoffkonzentration im (ggf. trockenen) Prozessgas aufgrund der Mittelung des Prozessgases aus zahlreichen Reaktionsrohren (typischerweise mehrere 1.000 bis mehrere 10.000) nur bedingt zur frühzeitigen Erkennung eines thermisch instabilen Betriebszustandes heranziehen. Dafür ist, wie ebenfalls oben erwähnt, die Temperatur im Katalysatorbett von einer repräsentativen Anzahl an mit entlang des Katalysatorbetts befindlicher Temperaturmessstellen versehener Reaktionsrohre besser geeignet.

Allerdings sei hier darauf hingewiesen, dass die beschriebenen Maßnahmen zur Deeskalation eines thermisch instabilen Betriebszustandes, auch im Falle eines großtechnischen Reaktors mit zahlreichen Rohren, genau auch die oben beschriebenen und im Beispiel 3 aufgezeigten Effekte auf die Sauerstoffkonzentration im Prozessgasstrom haben, da die aufgeführten Maßnahmen Änderungen im gesamten Reaktionseinsatzstrom darstellen und daher den gesamten Reaktor und daher auch jedes einzelne Reaktionsrohr gleich betreffen. Daher kann zur Beurteilung, ob ein thermisch instabiler Betriebszustand durch getroffene Deeskalationsmaßnahmen behoben wurde, der Trend der Kurve der Sauerstoffkonzentration im (ggf. trockenen) Prozessgasstrom herangezogen werden.

Es ist bei Ausführung in einem großkommerziellen Reaktor möglich, insbesondere bei den höheren Deeskalationsstufen, verschärfte Detektionskriterien anzuwenden, insb. im Hinblick auf die jeweilige Anzahl der instrumentierten Reaktionsrohre, die einen lokalen instabilen Betriebszustand aufweisen. So wäre es beispielsweise denkbar, dass für Auslösen der Deeskalationsstufe (c) mindestens 5%, 10% oder 30% der instrumentierten Rohre bzw. der in Flussrichtung axialen Temperaturprofile einen thermisch instabilen Betriebszustand auch nach Anwendung der vorhergehenden Deeskalationsstufen (a) und (b) anzeigen. In diesem Fall kann, wie oben beschrieben, auch eine Kombination lokaler Temperaturmessungen und der globalen Sauerstoffmessung am Austritt für die Festlegung der Deeskalationskriterien angewandt werden.

Nachfolgend wird im Zusammenhang mit Beispielen, die den technischen Hintergrund und Ausgestaltungen der Erfindung veranschaulichen, nochmals auf die Ursachen für ein thermisch instabiles Verhalten eingegangen.

Wie oben erwähnt, ist ein Ziel der vorliegenden Erfindung ein Verfahren aufzuzeigen, mit dem ein thermisch instabiles Verhalten oder ein beginnendes thermisches Durchgehen eines Rohrbündel-Festbettreaktors oder von Teilbereichen eines Rohrbündel-Festbettreaktors oder einzelner Rohre eines Rohrbündel-Festbettreaktors erkannt werden kann.

Ein thermisch instabiles Verhalten bis hin zum thermischen Durchgehen eines gekühlten Festbett-Rohrbündelreaktors für die Oxidation von Kohlenwasserstoffen oder eines Teilbereichs eines gekühlten Rohrbündelreaktors oder eines einzelnen Rohres eines gekühlten Festbett-Rohrbündelreaktors kann durch verschiedene Betriebsstörungen verursacht werden, von denen hier einige beispielhaft aufgeführt sind (es sei darauf hingewiesen, dass diese Aufzählung nicht abschließend ist, ferner kann grundsätzlich auch durch eine Kombination verschiedener Betriebsstörungen ein thermisch instabiles Verhalten verursacht werden):
- Zu geringe Wärmeabfuhr aufgrund zu geringer Kühlleistung, z.B. durch:
   - Störung im Dampfkreislauf, der in einem großtechnischen Reaktor zur Wärmeabfuhr bzw. Kühlung des Kühlmittels verwendet wird
   - Störung in der Umwälzung des Kühlmittels, z.B. zu geringer Kühlmittelfluss durch den Reaktor
- Erhöhung der Verweildauer des Reaktionsgemischs im Reaktor durch:
   - verringerten Abfluss von Reaktionsprodukten aus dem Reaktor bei gleichbleibendem Zufluss des Reaktionseinsatzes aufgrund einer Verlegung bzw. Verstopfung des Prozessgaswegs stromab des Reaktors (dies entspricht einer unbeabsichtigten Druckerhöhung, die mit einer erhöhten Verweilzeit der Reaktanden einhergeht)
   - verringerten Zufluss des Reaktionseinsatzes bei konstantem Druck z.B. durch plötzlichen, teilweisen oder vollständigen Wegfall einer oder mehrerer Komponenten (im Falle der ODHE von Ethan, Sauerstoff und/oder Wasser) des Reaktionseinsatzes oder eines Teils des gesamten Reaktionseinsatzgemischs
- Ungünstige Betriebsbedingungen in deren Folge es zu Akkumulationen von reaktiven Zwischenverbindungen im Katalysatorbett kommt und diese plötzlich abreagieren können
- Zu schnelle Erhöhung der Kühlmitteltemperatur während des Anfahrens oder des Wiederanfahrens
- Zu hohe Kühlmitteltemperatur beim Wechsel auf einen anderen Betriebspunkt

Während ausgedehnter Messkampagnen in einem Pilotreaktor wurden verschiedene thermisch instabile Reaktorverhaltensweisen sowie beginnendes oder nahezu thermisches Durchgehen und vollständiges thermisches Durchgehen bei der oxidativen Dehydrierung von Ethan beobachtet. Als Ursache konnte dabei ein großer Teil der oben angegebenen Betriebsstörungen ermittelt werden (ursächlich dabei war meist nur eine der o.g. Störungen, also keine Kombination von Störungen).

Nachfolgend sei thermisch instabiles Verhalten an zwei Beispielen gezeigt.

Die hier angegebenen Beispiele für thermisch instabiles Verhalten wurden während ausgedehnter Messkampagnen in einem Pilotreaktor beobachtet.

Der verwendete Pilotreaktor ist ein mit einer Salzschmelze gekühlter Festbettreaktor. Es handelt sich dabei um den gleichen Pilotreaktor, mit dem auch beispielsweise die in der WO 2019/243480 A1 beschriebenen Ergebnisse erzielt wurden. Der Pilotreaktor ist als ein Rohr-in-Rohr Reaktor ausgeführt, wobei das Innenrohr mit dem KatalysatorFestbett befüllt ist (Reaktionsraum). Zwischen der Wandung des Reaktionsraumes und dem Außenrohr befindet sich der Kühlmittelraum, d.h. dieser Raum wird mit dem Kühlmittel, hier mit einer flüssigen Salzschmelze, im Gegenstrom zur Flussrichtung des Reaktionseinsatzstromes durchströmt. Bei der Salzschmelze handelt es sich um eine Mischung aus Natriumnitrit, Natriumnitrat sowie Kaliumnitrat. Die Dimensionen (d.h. Länge, Innendurchmesser und Wandstärke) des Reaktionsraumes des Pilotreaktors entsprechen den typischen Dimensionen eines einzelnen Reaktionsrohrs aus einem typischen kommerziellen (großtechnischen) Rohrbündelreaktor. Somit ist der Pilotreaktor als ein echtes Abbild einer großtechnischen Anlage anzusehen (also Scale-up vom Labormaßstab), da sich in diesem Pilotreaktor aufgrund seiner Geometrie die gleichen Verhältnisse (Strömungsfeld, Temperatur-bzw. Temperaturgradienten, Druckgradienten etc.) wie in einem technischen Rohrbündelreaktor einstellen und sich somit die Reaktion unter realen technischen Bedingungen testen lässt. Der Reaktor ist mit einem verschiebbaren 8-fach Thermoelement ausgestattet, das sich innerhalb eines Schutzrohres in der axialen Mitte des Katalysators befindet. Damit kann das gesamte Temperaturprofil des Reaktors gemessen werden oder gleichzeitig die Temperaturverläufe an acht Positionen im Katalysatorbett bei konstanter Stellung des Thermoelements.

In den verschiedenen Messkampagnen wurde der Reaktor mit unterschiedlichen Katalysatorbetten gefüllt. Dabei wurde immer das gleiche Katalysatorgrundmaterial, ein MoVNbTe-Mischoxid, verwendet. Der Katalysator wurde als Formkörper (Ringe) eingefüllt. Zur Herstellung der Formkörper wurde das Katalysatorgrundmaterial mit Hilfe eines Siliciumoxid-basierten Binders zu Katalystorformkörpern verpresst. Details zu Katalysatorherstellung finden sich beispielsweise in der DE 10 2017 000 861 A1.

In einem ersten Beispiel wurde ein instabiler Betrieb durch ungünstige Reaktionsbedingungen provoziert. Der Pilotreaktor wurde für den Testbetrieb mit einem ca. 6,6 m langen einlagigen Katalysatorbett gefüllt. Die dafür verwendeten Katalysatorringe waren mit der für diese Binderart geringsten möglichen Menge an Binder hergestellt worden, um die Verdünnung durch den Binder minimal zu halten. Stromauf und stromab der Katalysatorschicht befand sich jeweils ein Bett aus Inertmaterial, ebenfalls in Ringen nahezu der gleichen Größe wie die Katalysatorformkörper. Die eingefüllte Katalysatormasse (umfassend die Masse der Aktivkomponente und des Binders) betrug 3,03 kg. Die Betriebsbedingungen wurden während der gesamten Versuchskampagne von ca. 1000 h innerhalb folgender Fenster variiert:
- GHSV (Gesamtgasstrom des Reaktionseinsatzes inkl. aller Komponenten per Volumen des Katalysatorbetts): 1000-1700 h⁻¹ (bzw. (Nm³/h)_{Gas}/m³_{Katalysator})
- Salzbadtemperatur: 290 bis 320 °C
- Systemdruck: 3,0 bis 4,5 bar abs.
- Wasser-Ethan-Verhältnis im Reaktionseinsatz: 0,25 bis 0,7 mol/mol
- Sauerstoff-Ethan-Verhältnis im Reaktionseinsatz: 0,3 bis 0,4 mol/mol
- Anteil Ethan im Reaktionseinsatz: 48 bis 65 mol%
- Oxidationsmittel: Sauerstoff

Unter diesen Bedingungen wurden folgende Ergebnisse erzielt:
- Selektivität zu Ethylen: 78 bis 84 mol%
- Selektivität zu Essigsäure: 11 bis 16 mol%
- Selektivität zu Kohlenmonoxid: 2,7 bis 3,2 mol%
- Selektivität zu Kohlendioxid: 1,4-1,6 mol%

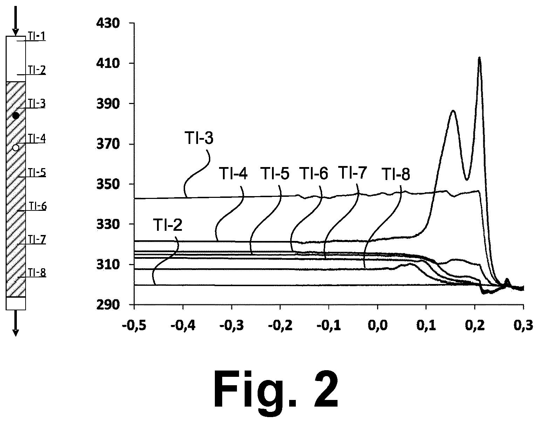

Bei erhöhtem Druck von 4,5 bar abs. und einem geringen Wasseranteil im Reaktionseinsatz (Wasser-Ethan-Verhältnis von mehr als 0,25 mol/mol) zeigte sich dabei ein thermisch instabiles Verhalten, das in seiner Folge zu einem thermischen Durchgehen führte. Dies ist in Figur 2 veranschaulicht.

Der linke Teil der Figur 2 zeigt dabei eine schematische Darstellung des zuvor erläuterten Pilotreaktors, ausgestattet mit einem 8-fach Thermoelement (TI-1 bis TI-8) in der Katalysatorschüttung. Die ungefähre Positionierung der Thermoelemente während des Versuchs kann der schematischen Abbildung entnommen werden. Ein ausgefüllter Punkt bezeichnet das Temperaturmaximum (Hotspot) in der schraffiert dargestellten Katalysatorschüttung während des Normalbetriebs. Die Messstelle TI-3 befand sich bei dieser Stellung des verschiebbaren Thermoelements in der Nähe des Hotspots des Normalbetriebs. Ein nicht ausgefüllter Punkt bezeichnet den globalen Hotspot während des thermischen Durchgehens.

Der rechte Teil der Figur 2 zeigt ein Diagramm, in dem die Temperaturverläufe der einzelnen Messpunkte (TI-2 bis TI-8 gemäß linkem Teil) in °C (Vertikalachse) gegenüber einer Zeit in h (Horizontalachse) aufgetragen sind Aufgrund der Positionierung war die Temperatur an TI-1 deutlich niedriger als 290 °C, daher ist TI-1 in der Grafik nicht dargestellt. Der Zeitpunkt "0" auf der Horizontalachse bezeichnet den ungefähren Startzeitpunkt des thermisch instabilen Verhaltens, d.h verstärkter Anstieg von TI-4 und gleichzeitiges Sinken von TI-5 und TI-6.

Anhand der Temperaturverläufe aus Figur 2 ist folgendes zu erkennen:
(1) Die thermisch instabile Zone innerhalb des Katalysatorbetts bildete sich bei konstantem (stationären) Betrieb stromab des eigentlichen Hotspots während des Normalbetriebs.
(2) Im thermisch instabilen Betrieb verschiebt sich der Hotspot entlang der Flussrichtung im Katalysatorbett, oder es bildete sich stromab des Hotspots aus dem Normalbetrieb ein weiterer globaler Hotspot
(3) Die Temperatur bei T-2 und T-3 im Katalysatorbett stromauf der gebildeten thermisch instabilen (heißesten) Zone im Katalysatorbett (die sich in der Nähe von TI-4 in Figur 2 befindet) verläuft nahezu konstant bzw. steigt minimal an (aufgrund der finalen Stabilisierung der Kühlmitteltemperatur). Die Temperaturen unmittelbar stromab der thermisch instabilen Zone (insbesondere TI-5 und TI-6) sinken stetig und nähern sich der Kühlmitteltemperatur (entspricht der Temperatur im Inertbett TI-2 kurz vor dem Katalysatorbett) an.
(4) Der thermisch instabile Betrieb beginnt zum Zeitpunkt 0 (vgl. Figur 2), d.h. zu dem Zeitpunkt, an dem die Temperatur nahe des globalen Hotspots zu steigen beginnt während gleichzeitig die Temperaturen stromab des globalen Hotspots zu sinken beginnen. Ohne an die Theorie gebunden zu sein, kann das Sinken der Temperaturen stromab des globalen Hotspots ist mit einem Sauerstoffmangel und somit einem Stopp der Oxidationsreaktion zu erklären sein.
(5) Beim thermischen Durchgehen (ab Zeitpunkt ca. 0,1 h in Figur 2) steigt die Temperatur im globalen Hotspot sehr schnell und steil an, entsprechend sinken die Temperaturen stromab des Hotspots auch schnell auf nahezu Kühlmittel-(Salzschmelze-)Niveau. Im vorliegenden Fall ist eine kurzfristige Erholungsphase (Sinken der Temperatur am Hotspot bei ca. 0,18 h) zu beobachten, bevor das Durchgehen bei ca. 0,20 h nicht mehr zu stoppen war. Bei ca. 0,21 h erfolgte eine automatische Sicherheitsabschaltung

Es sei hier noch einmal ausdrücklich erwähnt, dass das beobachtete thermisch instabile Verhalten auf die gewählten speziellen Versuchsbedingungen zurückzuführen ist, und somit auch eine Grenze für ein Verfahrensbetriebsfenster darstellt. In der gesamten vorangegangenen Zeitspanne von nahezu 1000 Betriebsstunden mit dieser Reaktorfüllung wurde kein derartiger instabiler Betrieb beobachtet.

Gemäß einem zweiten Beispiel wurde ein instabiler Betrieb durch eine Erhöhung der Verweildauer im Reaktor provoziert. Für den Testbetrieb wurde der Pilotreaktor mit einem hinsichtlich der katalytischen Aktivität dreistufigen Katalysatorbett gefüllt. Dabei war das katalytisch aktive Grundmaterial für jede Stufe exakt das gleiche. Das Bett war dabei so angeordnet, dass die katalytische Aktivität in Flussrichtung des Reaktionseinsatzstromes zunahm. Die unterschiedliche Aktivitätsabstufung wurde (wie auch in der WO 2019/243480 A1) durch Katalysatorformkörper (Ringe) mit unterschiedlich hoher Beimengung an Binder, der zur Formung der Formkörper benötigt wird, zum exakt gleichen katalytisch aktiven Grundmaterial erreicht. Der Binder fungiert also gleichzeitig auch als Verdünnungsmittel des aktiven Katalysatormaterials. Jede Katalysatorschicht wies die gleiche Höhe und somit das gleiche Volumen auf. Die Gesamtlänge des Katalysatorbetts betrug 6,6 m. Die Masse an eingefülltem Katalysator (inkl. Binderanteil) betrug 2,62 kg. Stromauf und stromab des dreistufigen Katalysatorbetts befand sich jeweils eine Schüttung aus Inertmaterial gleicher Form und Größe wie die Katalysatorformkörper.

Der Pilotreaktor wurde sodann mit dieser Katalysatorfüllung über einen Zeitraum von ca. 1.700 h mit einem Reaktionseinsatzstrom im Wesentlichen bestehend aus Ethan, Sauerstoff sowie Wasser(dampf) betrieben. Das molare Verhältnis von Ethan zu Sauerstoff zu Wasser(dampf) im Reaktionseinsatzstrom betrug 59 zu 24 zu 17. Der Druck am Reaktoreintritt betrug 3,81 bar abs., die GHSV betrug 1.088 h⁻¹ (bzw. (Nm³/h)_{Gas}/m³_{Katalysator}). Über den gesamten Testzeitraum war eine gleichbleibende, stabile und sehr gute Reaktor- bzw. Katalysatorperformance hinsichtlich Ethanumsatz und Selektivitäten zu den gewünschten kommerziellen Wertprodukten Ethylen und Essigsäure zu beobachten. Der Ethanumsatz lag bei über 52%, die Selektivität zu Ethylen bei über 82% und die Selektivität zu Essigsäure bei ca. 12%, d.h. einer Gesamtselektivität zu kommerziellen Wertprodukten von mehr als 94%.

Nach ca. 1.200 Betriebsstunden wurde ein thermisch instabiles Verhalten (vgl. Figur 3) aufgrund einer Erhöhung der Verweildauer im Reaktor induziert. Die Erhöhung der Verweildauer wurde im vorliegenden Fall durch eine Erhöhung des Systemdrucks hervorgerufen, wodurch das durch die Reaktion entstandene Prozessgas in geringerer Menge abgeführt werden konnte.

Der linke Teil der Figur 3 zeigt dabei eine schematische Darstellung des zuvor erläuterten Pilotreaktors, ausgestattet mit einem 8-fach Thermoelement (TI-1 bis TI-8) in der Katalysatorschüttung, die hier aus unterschiedlich schraffiert dargestellten Reaktionszonen besteht, deren Aktivität in Strömungsrichtung zunimmt (vgl. das keilförmige Element links). Die ungefähre Positionierung der Thermoelemente während des Versuchs kann der schematischen Abbildung entnommen werden. Ausgefüllte Punkte bezeichnen die Temperaturmaxima (Hotspots) in den jeweiligen Katalysatorschüttungen während des Normalbetriebs. Der Hotspot der zweiten Katalysatorschicht war zugleich auch der globale Hotspot (vgl. Temperaturen von TI-4, TI-5 und TI-6). Die Messstelle TI-1 befindet sich bei dieser Positionierung des verschiebbaren Thermoelements ca. in der Mitte der unschraffiert dargestellten Interschicht stromauf des Katalysatorbetts. Die Inertschicht ist zugleich die Vorheizzone, in der der Reaktionseinsatzstrom aufgrund der zirkulierenden Salzschmelze auf die Starttemperatur (entspricht Salzbadtemperatur) aufgeheizt wird.

Der rechte Teil der Figur 3 zeigt oben ein Diagramm, in dem die Temperaturverläufe der einzelnen Messpunkte (TI-2 bis TI-8 gemäß linkem Teil) in °C (Vertikalachse) gegenüber einer Zeit in h (Horizontalachse des unteren und zugleich oberen Diagramms) aufgetragen sind. Der Zeitpunkt "0" auf der Horizontalachse bezeichnet den ungefähren Startpunkt des thermisch instabilen Verhaltens, d.h verstärkter Anstieg von TI-5 und gleichzeitiges Absinken anderer. Ab Zeitpunkt ca. 0,32 h wurde die Erhöhung des Systemdrucks beendet. Im rechten Teil der Figur 3 ist unten die Konzentration an Sauerstoff im trockenen Prozessgas stromab des Reaktors in Volumenprozent (Vertikalachse) veranschaulicht.

Dieses thermisch instabile Verhalten wies die folgenden Merkmale auf:
(1) Die thermische Instabilität entwickelte sich am oder zumindest in der Nähe des im Normalbetrieb befindlichen lokalen Hotspots in der zweiten Katalysator- bzw. Reaktionszone (TI-5 war in dieser Position des verschiebbaren Thermoelements die heißeste Temperaturmessstelle und befand sich nur wenig stromab des lokalen Temperaturmaximums, vgl. schematische Reaktordarstellung in Figur 3 links). Dieser lokale Hotspot war im Normalbetrieb des Reaktors auch zugleich der globale Hotspot im gesamten Katalysatorbett.
(2) Zu Beginn des thermisch instabilen Verhaltens (Zeitpunkt "0" in Figur 3) steigen, analog zum ersten Beispiel, die Temperaturen aller Messstellen (TI-2 bis TI-5 in Abb. 3) im Katalysatorbett stromauf bis nahe/am globalen Hotspot an, während die Temperaturen aller Messstellen stromab des globalen Hotspots (TI-6 bis TI-8 in Abb. 3) bis auf nahezu Inertbett- bzw. Salzbadtemperatur (entspricht TI-1 in Abb. 3) abfallen.
(3) Es ergibt sich ferner ein deutlicher Abfall der Sauerstoffkonzentration im trockenen Prozessgas (d.h. nach Kondensation und Abtrennung des Prozesskondensats) stromab des Reaktors mit Beginn des thermisch instabilen Verhaltens (Zeitpunkt "0") aufgrund des nahezu vollständigen Sauerstoffverbrauchs im thermisch instabilen Bereich (vgl. Bemerkung oben). Somit ist das Reaktions/Prozessgas im Katalysatorbett stromab der thermisch instabilen Zone im Katalysatorbett/Reaktor stark an Sauerstoff verarmt oder sogar sauerstofffrei.

Anhand eines dritten Beispiels wurden Deeskalationsmethoden von thermisch instabilen Betriebszuständen mit dem oben beschriebenen Pilotreaktor mit der oben beschriebenen dreilagigen Katalysatorbettfüllung (vgl. zweites Beispiel, Figur 3) durchgeführt. Der Reaktionseinsatzstrom bestand im Wesentlichen aus Ethan, Sauerstoff sowie Wasser(dampf). Das molare Verhältnis von Ethan : Sauerstoff: Wasser(dampf) im Reaktionseinsatzstrom betrug für die Standard- bzw. Kontrolleinstellungen 59 zu 24 zu 17. Der Druck am Reaktoreintritt betrug 3,81 bar abs., die Salzbadtemperatur betrug 318,8 °C. Die übrigen Bedingungen bzw. Variationen sind nachfolgender Tabelle 1 zu entnehmen.

**Tabelle 1**

| **Nr.** | **Variation** | **Bedingungen bzw. geänderte Bedingungen** | **Beobachtung/Ergebnis** |
|---|---|---|---|
| 0 | Standard bzw. Kontrolle | - GHSV = 1093 (Nm³/h)_{Gas}/m³_{Katalysator} | - Konz. O₂*) = 0,62 mol% |
| | | - 02/C2H6 = 0,41 mol/mol | |
| | | - H2O/C2H6 = 0,29 mol/mol | |
| 1 | Erhöhung Ethanfluss um 10% | - GHSV gegenüber Nr. 0 um 5,4% erhöht | - Konz. O₂*) = 0,69 mol% |
| | | - 02/C2H6 = 0,37 mol/mol | |
| | | - H2O/C2H6 = 0,26 mol/mol | |
| 2 | Erhöhung Ethanfluss um 30% | - GHSV gegenüber Nr. 0 um 16,6% erhöht | - Konz. O₂*) = 1,03 mol% |
| | | - 02/C2H6 = 0,32 mol/mol | |
| | | - H2O/C2H6 = 0,23 mol/mol | |
| 3 | Erhöhung | - GHSV gegenüber Nr. 0 um 50% erhöht | - Konz. O₂*) = 1,36 mol% |
| | Ethanfluss um 50% | - 02/C2H6 = 0,27 mol/mol | - Wasser-Ethan-Verhältnis im Reaktionseinsatz zu niedrig zur Aufrechterhaltung der Katalysatoraktivität (stetig sinkende Temperaturen über den Zeitraum von Nr. 3) und somit Verstärkung des Deeskalierenden Effekts |
| | | - H2O/C2H6 = 0,19 mol/mol | |
| 4 | Erhöhung Ethan- und Sauerstofffluss um jeweils 30% | - GHSV gegenüber Nr. 0 um 23,2% erhöht | - Konz. O₂*) = 4,7 mol% |
| | | - H2O/C2H6 = 0,23 mol/mol | - Starker Temperaturanstieg im Katalysatorbett im Bereich relativer Sauerstoffarmut bzw. stromab einer thermisch instabilen Zone (TI-7 und TI-8, insbesondere TI-7 in Abb. 4) |
| 5 | Zudosierung von Stickstoff als Inertgas | - GHSV gegenüber Nr. 0 um 12% erhöht | - Konz. O₂*) > 3,2 mol% |
| 6 | Erhöhte Zufuhr von Stickstoff | - GHSV gegenüber Nr. 0 um 20% erhöht | - Konz. O₂*) ≈ 4,8 mol% |

| | | | |
|---|---|---|---|
| *) Mittlere Sauerstoffkonzentration im trockenen Prozessgas stromab des Reaktors | | | |

In Figur 4 sind die in Tabelle 1 veranschaulichten Maßnahmen (der Balken oberhalb des rechten Teils der Figur 4 entspricht der Numerierung in Tabellenspalte 2) zur stufenweisen Deeskalation im Falle von thermisch instabilen Reaktorverhalten oder beginnendem thermischen Durchgehen veranschaulicht. Mit "0" bezeichnete Phasen sind also die Standardbedingungen bzw. die Kontrollbedingungen, d.h. es erfolgt eine Rückkehr zu den Standard-Versuchsbedingungen nach jeder der Variationen 1 bis 4.

Im linken Teil der Figur 4 ist wiederum der Pilotreaktor schematisch gezeigt, der mit einem 8-fach Thermoelement (TI-1 bis TI-8) in der Katalysatorschüttung ausgestattet ist. Die ungefähre Positionierung der Thermoelemente während des Versuchs kann der schematischen Abbildung entnommen werden. Ausgefüllte Punkte stellen Temperaturmaxima (Hotspots) in der jeweiligen Katalysatorschicht während des Normalbetriebs, d.h. während der Standard/Kontrolleinstellungen dar.

Im rechten Teil der Figur 4 ist oben ein Diagramm veranschaulicht, in dem die Temperaturverläufe der einzelnen Messpunkte (TI-2 bis TI-8 gemäß linkem Teil) in °C (Vertikalachse) gegenüber einer Zeit in h (Horizontalachse der beiden dargestellten Diagramme) in den darüber angegebenen Zeitabschnitten (vgl. Tabellenspalte 1 der Tabelle 1) aufgetragen sind. Der Zeitpunkt "0" auf der Horizontalachse bezeichnet den Zeitpunkt des erstmaligen Abweichens von den Standardbedingungen. Im rechten Teil der Figur 4 sind ferner unten die Konzentration an Sauerstoff im trockenen Prozessgas stromab des Reaktors mit 401 (in Volumenprozent auf der linken Vertikalachse) und der Reaktordruck mit 402 (in bar abs. auf der rechten Vertikalachse) veranschaulicht. Die Kühlmitteltemperatur (Salzbadtemperatur) betrug für den gesamten Versuch ca. 318,8 °C. Die kurzeitigen negativen oder positiven Spitzen in der Temperatur TI-8 und der Sauerstoffkonzentration 401 sind die Folge von Druckstößen beim Umstellen auf neue Versuchsbedingungen, z.B. von Kontrollversuch 0 auf Versuch Nr. 2.

Wie mehrfach erwähnt, ermöglicht die Erfindung die Erhöhung der Betriebssicherheit durch automatisierte Überwachung des thermischen Betriebszustands eines katalytischen Festbettreaktors, wobei dieser Reaktor ein Rohrbündelreaktor sein kann. Gegebenenfalls kann auf dieser Grundlage ein automatisiertes Einleiten von Maßnahmen zum Deeskalieren von thermisch instabilen Betriebszuständen erfolgen, wobei diese Maßnahmen so gestaltet sind, dass ein weiterer Produktionsbetrieb ggf. bis zu Beseitigung der den thermisch instabilen Betriebszustand hervorrufenden Betriebsstörung aufrechterhalten werden kann. Aufgrund der weitgehenden Automatisierung Erhöhung der Anlagenbetriebssicherheit und Anlagenverfügbarkeit (Produktionsbetrieb) erfolgen. Dadurch kann insgesamt eine höhere Produktivität und/oder Effizienz eines entsprechenden Verfahrens bzw. einer entsprechenden Anlage erreicht werden.

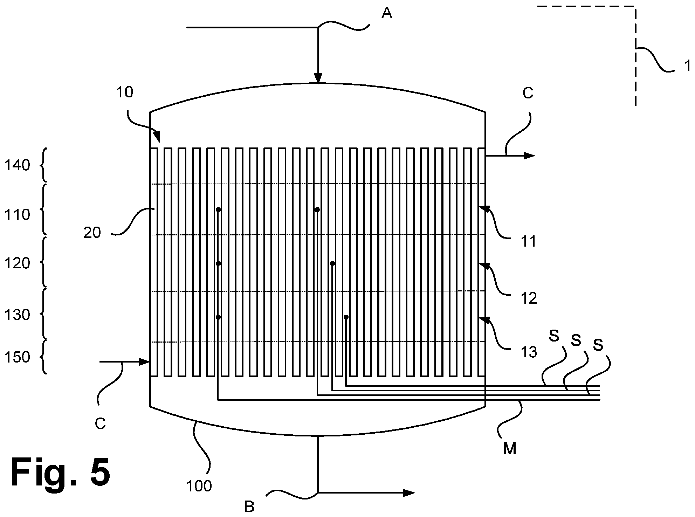

Eine Anlage zur Herstellung von Olefinen gemäß einer Ausführungsform der Erfindung weist, wie erwähnt, einen Rohrbündelreaktor 100 auf, wie er in Figur 5 veranschaulicht ist, oder mehrere solcher Rohrbündelreaktoren, wobei eine übergeordnete Anlage hier mit 1 angedeutet ist. Hierbei wird dem im dargestellten Beispiel einen Rohrbündelreaktor 100 ein Ethan enthaltendes, auf beliebige Weise gewonnenes Einsatzgemisch A zugeführt wird. Das Einsatzgemisch A kann beispielsweise einer nicht dargestellten Rektifikationseinheit entnommene Kohlenwasserstoffe enthalten. Das Einsatzgemisch A kann auch beispielsweise vorgewärmt und auf andere Weise aufbereitet werden. Das Einsatzgemisch A kann bereits Sauerstoff und ggf. einen Reaktionsmoderator wie Wasserdampf enthalten, entsprechende Medien können jedoch auch stromauf oder in dem Rohrbündelreaktor 100 zugegeben werden, wie nicht gesondert dargestellt. Dem Rohrbündelreaktor 100 wird ein Produktgemisch B entnommen.

Der Reaktor 100, der in Figur 5 im Detail dargestellt ist, weist eine Vielzahl parallel angeordneter Reaktionsrohre 10 auf (nur zum Teil bezeichnet), die durch eine Vorwärmzone 140 und danach durch mehrere, im dargestellten Beispiel drei, Reaktionszonen 110, 120, 130 verlaufen. Stromab kann eine Nachreaktionszone 150 vorhanden sein. Die Reaktionsrohre 10 sind von einem Mantelbereich 20 umgeben, durch die im Beispiel ein Kühlmittel C der erläuterten Art geführt ist. Die Darstellung ist stark vereinfacht, da, wie erwähnt, die Reaktionsrohre 10 ggf. unter Verwendung mehrerer, die Reaktionsrohre 10 umfließender Kühlmedien gekühlt werden können oder unterschiedliche Rohrabschnitte unter Verwendung unterschiedlicher Kühlmedien, desselben Kühlmediums in unterschiedlichen Kühlmedienkreisläufen, und/oder desselben oder unterschiedlicher Kühlmedien in unterschiedlichen oder gleichen Strömungsrichtungen gekühlt werden können.

Nach der Einspeisung in den Rohrbündelreaktor wird das Einsatzgemisch A in geeigneter Weise auf einer Temperatur in einem ersten Temperaturbereich auf die Reaktionsrohre 10 verteilt. Die Reaktionsrohre weisen jeweils Katalysezonen 11, 12 und 13 auf, die in den Reaktionszonen 120, 130 und 140 liegen.

Eine katalytische Umsetzung erfolgt mittels der in den Reaktionsrohren 10 hintereinander angeordneten Katalysezonen 11, 12 und 13, die optional bereitgestellt werden können und dabei beispielsweise eine unterschiedliche Aktivität und/oder Selektivität aufweisen können. Die Parameter des Einsatzgemischs und der Reaktionsbedingungen wurden mehrfach erläutert.

Schematisch sind ferner ein Mehrpunkt-Temperaturfühler M in einem der Reaktionsrohre und drei Einzelpunkt-Temperaturfühler S in drei weiteren der Reaktionsrohre veranschaulicht. Die entsprechende Anzahl kann beliebig sein. Die Temperaturmessstellen der Temperaturfühler M, S sind in Form von ausgefüllten Punkten veranschaulicht und können insbesondere bei dem Mehrpunkt-Temperaturfühler M auch in beliebiger anderer Weise beabstandet und in beliebiger anderer Anzahl bereitgestellt sein.

## Patentansprüche

1. Verfahren zur Herstellung einer Zielverbindung, bei dem ein wenigstens eine Eduktverbindung in Form eines oder mehrerer Kohlenwasserstoffe enthaltendes Einsatzgemisch (A) gebildet, auf parallel angeordnete Reaktionsrohre (10) eines oder mehrerer Rohrbündelreaktoren (100) verteilt, und in den Reaktionsrohren (10) einer oxidativen katalytischen Umsetzung unterworfen wird, **dadurch gekennzeichnet, dass** Überwachungsmaßnahmen durchgeführt werden, die umfassen, auf Grundlage von Temperaturmessungen an mehreren Messstellen entlang eines oder mehrerer der Reaktionsrohre (10) jeweils zeitliche Temperaturverläufe zu ermitteln, auf Grundlage zumindest der Temperaturverläufe eine Stabilitätsbewertung der katalytischen Umsetzung vorzunehmen, und, wenn auf Grundlage der Stabilitätsbewertung ein instabiler Zustand der katalytischen Umsetzung als eingetreten oder wahrscheinlich eintretend bestimmt wird, Stabilisierungsmaßnahmen einzuleiten.

2. Verfahren nach Anspruch 1, bei dem die Temperaturmessungen eine Vielzahl von Temperaturmessungen in einem Reaktionsrohr (10) oder in jedem von mehreren Reaktionsrohren (10) umfassen, die unter Verwendung eines oder mehrerer Mehrpunkt-Temperaturfühler (M) vorgenommen werden.

3. Verfahren nach Anspruch 1 oder nach Anspruch 2, bei dem die Temperaturmessungen eine Vielzahl von Temperaturmessungen in unterschiedlichen der Reaktionsrohre (10) umfassen, die unter Verwendung mehrerer Einzelpunkt-Temperaturfühler (S) vorgenommen werden.

4. Verfahren nach einem der vorstehenden Ansprüche, bei dem die Stabilitätsauswertung die vergleichende Bewertung der Temperaturverläufe an zumindest zwei der Messstellen, die in Strömungsrichtung hintereinander angeordnet sind, umfasst.

5. Verfahren nach einem der vorstehenden Ansprüche, bei dem der instabile Zustand der katalytischen Umsetzung als eingetreten oder wahrscheinlich eintretend erkannt wird, wenn an den zumindest zwei der Messtellen, die zur Beurteilung des in Flussrichtung axialen Temperaturprofils des Katalysatorbetts herangezogen werden, über mehr als einen vorgegebenen Zeitraum gegenläufige Temperaturtrends in mehr als einem vorgegebenen Umfang ermittelt werden.

6. Verfahren nach Anspruch 5, bei dem der vorgegebene Zeitraum mehr als 0,5 Minuten, mehr als 1 Minute, mehr als 2 Minuten und bis zu 3 Minuten beträgt, und/oder bei dem der vorgegebene Umfang mehr als 0,5%, mehr als 1%, mehr als 2%, mehr als 5% oder mehr als 10% eines Temperaturwerts zu Beginn des vorgegebenen Zeitraums beträgt.

7. Verfahren nach einem der vorstehenden Ansprüche, bei dem der instabile Zustand der katalytischen Umsetzung als nicht eingetreten oder nicht wahrscheinlich eintretend bestimmt wird, wenn an den zumindest allen der Messtellen, die zur Beurteilung des in Flussrichtung axialen Temperaturprofils des Katalysatorbetts herangezogen werden, gleichläufige Temperaturtrends oder konstante Temperaturen ermittelt werden.

8. Verfahren nach einem der vorstehenden Ansprüche, bei dem die Überwachungsmaßnahmen erst dann durchgeführt werden, wenn ein vorgegebener Zeitraum von insbesondere mehr als 1 Minute, 10 Minuten oder 1 Stunde seit einer letzten vorgenommenen Änderung von eine Reaktionsstabilität beeinflussenden Betriebssollwerten verstrichen ist.

9. Verfahren nach einem der vorstehenden Ansprüche, bei die Überwachungsmaßnahmen umfassen, auf Grundlage einer Sauerstoffmessung an einem stromabwärtigen Ende und/oder stromab eines oder mehrerer der Reaktionsrohre (10) ein oder mehrere Sauerstoffgehalte eines oder mehrerer Prozessgasströme zu ermitteln, wobei die Stabilitätsbewertung unter Berücksichtigung des oder der Sauerstoffgehalte durchgeführt wird.

10. Verfahren nach einem der vorstehenden Ansprüche, bei dem die Stabilisierungsmaßnahmen Maßnahmen unterschiedlicher Eingriffsschwere umfassen, wobei eine Maßnahme einer größeren Eingriffsschwere dann ergriffen wird, wenn eine Maßnahme geringerer Eingriffsschwere nach deren Durchführung als nicht ausreichend stabilisierend bestimmt und/oder als voraussichtlich nicht ausreichend stabilisierend betrachtet wird.

11. Verfahren nach Anspruch 10, bei dem die Maßnahmen unterschiedlicher Eingriffsschwere die folgenden, in ihrer Eingriffsschwere von (a) bis (c) zunehmenden Maßnahmen oder Kombinationen hiervon umfassen:
(a) schrittweise Erhöhung der stündlichen Raumgeschwindigkeit des Einsatzgemischs durch eine ausschließliche Erhöhung einer zeitlichen Menge der wenigstens einen Eduktverbindung,
(b) schrittweise Erhöhung der stündlichen Raumgeschwindigkeit des Einsatzgemischs durch eine Erhöhung der zeitlichen Menge wenigstens einer in dem Einsatzgemisch enthaltenen Inertkomponente,
(c) Unterbinden der Reaktandenzufuhr und Inertisierung des einen oder der mehreren Rohrbündelreaktoren (100).

12. Verfahren nach einem der vorstehenden Ansprüche, bei dem die oxidative katalytische Umsetzung unter Verwendung eines oder mehrerer, die Metalle Molybdän, Vanadium, Niob und optional Tellur enthaltender Katalysatoren durchgeführt wird

13. Verfahren nach einem der vorstehenden Ansprüche, bei dem Ethan als Einsatzverbindung verwendet wird und das oxidative katalytische Verfahren als oxidative Dehydrierung des Ethans durchgeführt wird.

14. Anlage zur Herstellung einer Zielverbindung mit einem oder mehreren, parallel angeordnete Reaktionsrohre (10) aufweisenden Rohrbündelreaktoren (100), die dafür eingerichtet ist, ein wenigstens eine Eduktverbindung in Form eines oder mehrerer Kohlenwasserstoffe enthaltendes Einsatzgemisch (A) zu bilden, auf Reaktionsrohre (10) des oder der Rohrbündelreaktoren (100) zu verteilen, und in den Reaktionsrohren (10) einer oxidativen katalytischen Umsetzung zu unterwerfen, **dadurch gekennzeichnet, dass** die Anlage dafür eingerichtet ist, Überwachungsmaßnahmen durchzuführen, die umfassen, auf Grundlage von Temperaturmessungen an mehreren Messstellen entlang eines oder mehrerer der Reaktionsrohre (10) jeweils zeitliche Temperaturverläufe zu ermitteln, auf Grundlage zumindest der Temperaturverläufe eine Stabilitätsbewertung der katalytischen Umsetzung vorzunehmen, und, wenn auf Grundlage der Stabilitätsbewertung ein instabiler Zustand der katalytischen Umsetzung als eingetreten oder wahrscheinlich eintretend bestimmt wird, Stabilisierungsmaßnahmen einzuleiten.
